# EUROPÄISCHE PATENTANMELDUNG

(11) **EP 2 702 935 A1**
(43) Veröffentlichungstag der Anmeldung: **05.03.2014**
(21) Anmeldenummer: 12006124.7
(22) Anmeldetag: 29.08.2012
(51) Int. Cl.: A61B 5/00, G01N 21/47, G01B 9/02, G06K 9/32

(54) **System und Verfahren zur optischen Kohärenztomographie sowie Positionierelement**

(71) Anmelder: Agfa HealthCare N.V., 2640 Mortsel (BE)
(72) Erfinder: Schorre, Wolfgang, 82515 Wolfratshausen (DE); Nebosis, Rainer, Dr., 81541 München (DE)
(74) Vertreter: Linsmeier, Josef

(57) **Zusammenfassung**

Die Erfindung betrifft ein System sowie ein Verfahren zur optischen Kohärenztomographie mit einem Interferometer und einem Sensorkopf, über welchen elektromagnetische Strahlung vom Interferometer an ein zu untersuchendes Objekt ausgegeben und vom Objekt reflektierte elektromagnetische Strahlung in das Interferometer zurück geführt werden kann, einem Positionierelement (3) zum Positionieren des Sensorkopfes relativ zu dem zu untersuchenden Objekt mit einer Auflage, welche am Objekt angeordnet und auf welcher der Sensorkopf platziert werden kann, und einem ersten Bereich (4), welcher für die vom Interferometer ausgegebene und vom Objekt reflektierte Strahlung im Wesentlichen durchlässig ist, und einem zweiten Bereich (5), dessen Durchlässigkeit für die vom Interferometer ausgegebene und/oder vom Objekt reflektierte Strahlung von der Durchlässigkeit des ersten Bereichs abweicht, einer Bilderzeugungseinrichtung zur Erzeugung eines oder mehrerer Bilder anhand der vom Objekt und/oder vom Positionierelement, insbesondere vom zweiten Bereich des Positionierelements, reflektierten elektromagnetischen Strahlung, und einer Steuerungseinrichtung zur Steuerung der Erzeugung und Wiedergabe der Bilder in der Weise, dass der Sensorkopf anhand der erzeugten und wiedergegebenen Bilder in eine gewünschte Position auf dem Positionierelement gebracht werden kann.

## Beschreibung

Die vorliegende Erfindung betrifft ein System und Verfahren zur optischen Kohärenztomographie sowie ein Positionierelement zum Positionieren eines Sensorkopfes relativ zu einem zu untersuchenden Objekt.

Die optische Kohärenztomographie (OCT) ist eine Methode, um lichtstreuende Proben in ihrem Inneren zu vermessen. Biologisches Gewebe ist aufgrund seiner lichtstreuenden Eigenschaften für die diagnostische Untersuchung mittels OCT besonders geeignet. Da die OCT mit relativ geringen Lichtintensitäten auskommt und die Wellenlängen des verwendeten Lichts zumeist in nahen Infrarotbereich (750 nm bis 1350 nm) liegen, stellt sie im Gegensatz zur ionisierenden Röntgendiagnostik für biologisches Gewebe keine Strahlenbelastung dar. Sie ist somit besonders für die Medizin von Bedeutung und grob vergleichbar mit der Ultraschalldiagnostik, wobei anstelle von Schall bei der OCT Licht verwendet wird. Die Laufzeiten des an unterschiedlichen Grenzschichten in der Probe reflektierten Lichts werden mit Hilfe eines Interferometers erfasst. Mit der OCT sind typischerweise um ein bis zwei Größenordnungen höhere Auflösungen als mit Ultraschall zu erreichen, jedoch ist die erzielbare Vermessungstiefe deutlich kleiner. Die gewonnenen Querschnittsbilder reichen aufgrund von optischer Streuung in der Regel nur bis zu einer Tiefe von wenigen Millimetern in das Gewebe hinein. Die derzeit wichtigsten Anwendungsbereiche der OCT liegen in der Ophthalmologie, der Dermatologie sowie der Krebsdiagnose. Es existieren allerdings auch einige nichtmedizinische Anwendungen, wie z.B. in der Werkstoffprüfung.

Bei medizinischen Anwendungen ist eine exakte Positionierung des Sensorkopfes eines OCT-Systems relativ zu dem zu untersuchenden Objekt von hoher Relevanz für die Zuverlässigkeit der bei einer Untersuchung gewonnenen diagnostischen Informationen. Gleichzeitig sollte die Handhabung möglichst einfach und zeitsparend gestaltet werden.

Es ist Aufgabe der vorliegenden Erfindung, ein System und Verfahren zur optischen Kohärenztomographie sowie ein Positionierelement zur Verwendung in einem solchen System bzw. Verfahren anzugeben, durch welches bei möglichst einfacher Handhabung eine zuverlässige und zeitsparende Untersuchung eines Objekts ermöglicht wird.

Diese Aufgabe wird durch das System, Verfahren bzw. Positionierelement gemäß den unabhängigen Ansprüchen gelöst.

Das erfindungsgemäße System zur optischen Kohärenztomographie weist auf:
- ein Interferometer und einen Sensorkopf, über welchen elektromagnetische Strahlung vom Interferometer an ein zu untersuchendes Objekt ausgegeben und vom Objekt reflektierte elektromagnetische Strahlung in das Interferometer zurück geführt werden kann,
- ein Positionierelement zum Positionieren des Sensorkopfes relativ zu dem zu untersuchenden Objekt mit einer Auflage, welche am Objekt angeordnet und auf welcher der Sensorkopf platziert werden kann, und einem ersten Bereich, welcher für die vom Interferometer ausgegebene und vom Objekt reflektierte Strahlung im Wesentlichen durchlässig ist, und einem zweiten Bereich, dessen Durchlässigkeit für die vom Interferometer ausgegebene und/oder vom Objekt reflektierte Strahlung von der Durchlässigkeit des ersten Bereichs abweicht,
- eine Bilderzeugungseinrichtung zur Erzeugung eines oder mehrerer Bilder anhand der vom Objekt und/oder vom Positionierelement, insbesondere vom zweiten Bereich des Positionierelements, reflektierten elektromagnetischen Strahlung,

- eine Anzeigeeinrichtung zur Wiedergabe der erzeugten Bilder und
- eine Steuerungseinrichtung zur Steuerung der Erzeugung und Wiedergabe der Bilder in der Weise, dass der Sensorkopf anhand der erzeugten und wiedergegebenen Bilder in eine gewünschte Position auf dem Positionierelement gebracht werden kann.

Beim erfindungsgemäßem Verfahren zur optischen Kohärenztomographie:
- wird elektromagnetische Strahlung von einem Interferometer über einen Sensorkopf an ein zu untersuchendes Objekt ausgegeben und vom Objekt reflektierte elektromagnetische Strahlung über den Sensorkopf in das Interferometer zurück geführt,
- wird ein Positionierelement zum Positionieren des Sensorkopfes relativ zu dem zu untersuchenden Objekt am Objekt angeordnet und der Sensorkopf auf der Auflage platziert, wobei die Auflage einen ersten Bereich, welcher für die vom Interferometer ausgegebene und vom Objekt reflektierte Strahlung im Wesentlichen durchlässig ist, und einem zweiten Bereich, dessen Durchlässigkeit für die vom Interferometer ausgegebene und/oder vom Objekt reflektierte Strahlung von der Durchlässigkeit des ersten Bereichs abweicht, aufweist,
- werden anhand der vom Objekt und/oder vom Positionierelement, insbesondere vom zweiten Bereich des Positionierelements, reflektierten elektromagnetischen Strahlung ein oder mehrere Bilder erzeugt und wiedergegeben und
- wird der Sensorkopf anhand der erzeugten und wiedergegebenen Bilder in eine gewünschte Position auf dem Positionierelement gebracht.

Das erfindungsgemäße Positionierelement zur Verwendung in einem System zur optischen Kohärenztomographie, wobei das System ein Interferometer und einen Sensorkopf aufweist, über welchen elektromagnetische Strahlung vom Interferometer an ein zu untersuchendes Objekt ausgegeben und vom Objekt reflektierte elektromagnetische Strahlung in das Interferometer zurück geführt werden kann, wobei das Positionierelement:
- ein Positionieren des Sensorkopfes relativ zu einem zu untersuchenden Objekt erlaubt und
- eine Auflage aufweist, welche am Objekt angeordnet werden kann und auf welcher der Sensorkopf platziert werden kann, wobei die Auflage einen ersten Bereich, welcher für die von einem Interferometer ausgegebene und vom Objekt reflektierte Strahlung im Wesentlichen durchlässig ist, und einem zweiten Bereich, dessen Durchlässigkeit für die vom Interferometer ausgegebene und/oder vom Objekt reflektierte Strahlung von der Durchlässigkeit des ersten Bereichs abweicht, aufweist.

Die Erfindung basiert auf dem Gedanken, zum Positionieren eines OCT-Sensorkopfes relativ zu dem zu untersuchenden Objekt ein Positionierelement zu verwenden, das mindestens zwei Bereiche mit unterschiedlichen optischen Eigenschaften, insbesondere unterschiedliche Durchlässigkeit und/oder Reflexivität für die vom Interferometer ausgegebene und/oder vom Objekt reflektierte elektromagnetische Strahlung, aufweist. Das Positionierelement wird hierbei am Objekt so angeordnet, dass sich der zu untersuchende Bereich des Objekts, beispielsweise eine Hautläsion, in einem ersten Bereich des Positionierelements befindet, welcher für die vom Interferometer ausgegebene und/oder vom Objekt reflektierte Strahlung im Wesentlichen durchlässig ist. Aufgrund der unterschiedlichen optischen Eigenschaften der beiden Bereiche des Positionierelements ist in den erfassten und angezeigten OCT-Bildern ein Übergang zwischen dem ersten und zweiten Bereich erkennbar, falls der Sensorkopf so auf das Positionierelement aufgesetzt worden ist, dass das Durchtrittsfenster des Sensorkopfes im Bereich des Übergangs zwischen dem ersten und zweiten Bereich zu liegen kommt. Eine Bedienperson, die einen solchen Übergang in den angezeigten OCT-Bildern erkennt, kann dann entscheiden, ob die Position des Sensorkopfes gegenüber der bisherigen Position zu verändern ist, um beispielsweise das Durchtrittsfenster möglichst mittig zum ersten Bereich zu platzieren und dadurch zu gewährleisten, dass einerseits OCT-Bilder exakt von dem zu untersuchenden Bereich des Objekts aufgenommen werden und andererseits der jeweils abgebildete Bereich der Probe eine optimale Größe hat. Insbesondere handelt es sich bei den hierbei erzeugten und wiedergegebenen OCT-Bildern um Echtzeitbilder, anhand welcher eine Positionierung einschließlich einer ggf. erforderlichen Positionsänderung des Sensorkopfes durch die Bedienperson zuverlässig und besonders komfortabel vorgenommen werden kann.

Insgesamt erlaubt die Erfindung eine exakte Positionierung des Sensorkopfes eines OCT-Systems relativ zu dem zu untersuchenden Objekt bei gleichzeitig einfacher und zeitsparender Handhabung.

Vorzugsweise ist der zweite Bereich für die vom Interferometer ausgegebene und/oder vom Objekt reflektierte Strahlung im Wesentlichen undurchlässig. Ein Übergang zwischen dem ersten Bereich, welcher für die vom Interferometer ausgegebene und/oder vom Objekt reflektierte Strahlung im Wesentlichen durchlässig ist, wird dadurch in den entsprechenden OCT-Bildern besonders deutlich sichtbar und erlaubt daher eine besonders exakte Positionierung.

Alternativ oder zusätzlich weist der zweite Bereich eine Struktur auf, deren optische Eigenschaften sich von den optischen Eigenschaften von Strukturen des Objekts unterscheiden. Werden mit dem OCT-System beispielsweise OCT-Bilder des menschlichen Hautorgans aufgenommen, so wird ein Positionierelement gewählt, dessen zweiter Bereich eine Struktur aufweist, die in den jeweiligen OCT-Bildern gut sichtbar von den zu erwartenden, typischen Strukturen des Hautorgans abweicht. Vorzugsweise weist die Struktur des zweiten Bereichs, insbesondere wiederkehrende, Strukturelemente auf, welche vom OCT-System zuverlässig aufgelöst werden können und insbesondere eine laterale Ausdehnung von etwa mindestens 3 µm haben. Eine solche Struktur des zweiten Bereichs ist mit hoher Zuverlässigkeit vom ersten Bereich, durch welchen hindurch der zu untersuchenden Bereich des Objekts abgebildet wird, in den jeweiligen OCT-Bildern zu unterscheiden, was die Positionierung ebenfalls besonders sicher und für den Bediener einfach macht.

Bei einer ebenfalls bevorzugten Ausführung ist die Auflage des Positionierelements biegsam ausgestaltet. Die Auflage kann sich hierbei insbesondere an Konturen des Objekts anpassen. Das Positionierelement lässt sich dadurch auch an gekrümmten Stellen des zu untersuchenden Objekts auf einfache Weise anbringen, wodurch gewährleistet wird, dass der erste Bereich des Positionierelements sicher auf dem zu untersuchenden Bereich des Objekts zu liegen kommt und dort sowohl während der Positionierung des Sensorkopfes als auch bei der eigentlichen Aufnahme von OCT-Bildern zu Diagnosezwecken verbleibt. Auch hierdurch wird bei einfacher Handhabung eine exakte Positionierung des Sensorkopfes relativ zu dem zu untersuchenden Objekt gewährleistet.

Vorzugsweise ist die Auflage des Positionierelements derart ausgestaltet, dass diese mit dem Objekt in Kontakt gebracht und insbesondere am Objekt fixiert werden kann. Insbesondere kann die Auflage lösbar am Objekt fixiert werden. Vorzugsweise ist die Auflage auf seiner dem Objekt zugewandten Unterseite mit mindestens einem Haftbereich versehen, welcher bei Kontakt mit dem Objekt eine lösbare Haftverbindung mit diesem eingeht. Beispielsweise kann der Haftbereich eine Schicht aus Haftklebstoff aufweisen, welche z.B. durch Aufbringen von Haftklebstoff direkt auf die Unterseite der Auflage oder aber auch durch Aufkleben eines Abschnittes eines doppelseitigen Klebebandes auf die Unterseite der Auflage realisiert werden kann. Vorzugsweise ist der Haftbereich, insbesondere die Haftklebstoffschicht, mit einer zusätzlichen Schutzschicht versehen, die unmittelbar vor der Verwendung des Positionierelements entfernt wird. Als Schutzschicht ist beispielsweise gewachstes oder silikonisiertes Schutzpapier besonders geeignet. Vorzugsweise ist nur ein Teilbereich der Unterseite der Auflage, insbesondere in der Umgebung des ersten Bereichs, mit einem Haftbereich versehen.

Aufgrund der Kontaktierung des Objekts bzw. der lösbaren Fixierung des Positionierelements am Objekt ist eine Positionierung des Sensorkopfes relativ zum Objekt, insbesondere zu dem zu untersuchenden Bereich des Objekts, besonders zuverlässig zu realisieren, da bei einer gegebenenfalls erforderlichen Positionsänderung des Sensorkopfes während des Positioniervorgangs ein Verrutschen des Positionierelements auf einfache und sichere Weise unterbunden wird. Darüber hinaus bleibt dadurch das Positionierelement auch während der eigentlichen Aufnahme von OCT-Bildern zu Diagnosezwecken mit hoher Zuverlässigkeit über dem zu untersuchenden Bereich des Objekts, so dass eine Aufnahme von OCT-Bildern exakt von dem ursprünglich ausgewählten Bereich des Objekts erfolgt.

Es ist ferner bevorzugt, dass der erste Bereich der Auflage als Durchbruch in der Auflage ausgebildet ist. Der erfindungsgemäße Unterschied der optischen Eigenschaften des ersten und zweiten Bereichs lässt sich auf diese Weise besonders einfach realisieren. Gleichzeitig wird dadurch eine besonders zuverlässige Positionierung des Sensorkopfes ermöglicht.

Bei einer weiteren bevorzugten Ausführung ist die Form und/oder Größe des ersten Bereichs der Auflage an die Form bzw. Größe eines Durchtrittsbereichs des Sensorkopfes, durch welchen die vom Interferometer ausgegebene und die vom Objekt reflektierte Strahlung hindurch treten kann, angepasst. Hat der Sensorkopf beispielsweise einen Durchtrittsbereich in Form eines im Wesentlichen kreisförmigen Fensters mit einem Durchmesser von etwa 2,5 mm, so ist der erste Bereich ebenfalls im Wesentlichen kreisförmig ausgestaltet mit einem Durchmesser von etwa 2,5 mm. Entsprechendes gilt für rechteckige und andere Formen des Durchtrittsbereichs, beispielsweise auch für rechteckige, insbesondere quadratische, Grundformen mit abgerundeten Ecken. Hierdurch wird einerseits eine exakte Positionierung des Sensorkopfes relativ zum Objekt und andererseits die Aufnahme von OCT-Bildern eines maximalen Ausschnitts aus dem Objekt ermöglicht.

Vorzugsweise sind am Positionierelement ein oder mehrere Markierungen vorgesehen, welche ein Positionieren des Sensorkopfes relativ zum Positionierelement, insbesondere relativ zum ersten und/oder zweiten Bereich der Auflage, unterstützen. Bei den Markierungen kann es sich beispielsweise um Linien und/oder Kreise und/oder kreisförmige Abschnitte handeln. Die Markerungen ermöglichen eine erste grobe Platzierung des Sensorkopfes, bevor dieser dann anschließend beim erfindungsgemäßen Positioniervorgang anhand der angezeigten OCT-Bilder ganz exakt relativ zum Objekt positioniert wird. Durch die Vorplatzierung des Sensorkopfes wird der Positioniervorgang selbst weiter vereinfacht und beschleunigt.

Die Markierungen sind vorzugsweise so gestaltet, dass sie äußeren Umrissen und/oder Formen des Sensorkopfes entsprechen. Weist das objektseitige Ende des Sensorkopfes beispielsweise einen kreisförmigen Auflagebereich auf, in dessen Mitte der Durchtrittsbereich in Form eines kreisförmigen Fensters angeordnet ist, so können als Markierung z.B. zwei konzentrische kreisförmige oder kreissektorförmige Ringe vorgesehen sein, deren Mittelpunkte mit dem Mittelpunkt des Auflagebereichs bzw. des kreisförmigen Fensters zusammenfallen. Der Durchmesser des inneren Rings der beiden Ringe entspricht vorzugsweise im Wesentlichen dem Außendurchmesser des Auflagebereichs des Sensorkopfes, während der äußere Ring einen etwas größeren Durchmesser als der innere Ring aufweist. Da das menschliche Auge bereits geringfügige Abweichungen von einer konzentrischen Platzierung des Auflagebereichs des Sensorkopfes erkennt, kann die Bedienperson mit Hilfe des äußeren Rings auf einfache und zuverlässige Weise eine sichere Vorplatzierung des Sensorkopfes vornehmen.

Es ist ferner bevorzugt, dass der zweite Bereich der Auflage eine Durchlässigkeit für die vom Interferometer ausgegebene bzw. vom Objekt reflektierte Strahlung von weniger als 10⁻⁴, insbesondere weniger als 10⁻⁶, aufweist. Da durch den zweiten Bereich der Auflage nur einen Anteil von weniger als 0,01 % bzw. weniger als 0,0001 % der auf den zweiten Bereich treffenden Strahlung hindurchtreten kann, ist der zweite Bereich in den aufgenommenen OCT-Bildern gegenüber dem ersten Bereich, welcher für die vom Objekt reflektierte Strahlung im Wesentlichen durchlässig ist, deutlich erkennbar, so dass der Sensorkopf mit hoher Präzision und Sicherheit exakt gegenüber dem Objekt positioniert werden kann. Es ist besonders bevorzugt, dass der zweite Bereich der Auflage eine Reflexionsschicht aufweist, welche die vom Interferometer ausgegebene Strahlung reflektiert. Insbesondere weist der zweite Bereich der Auflage, insbesondere die Reflexionsschicht, ein Reflexionsvermögen für die vom Interferometer ausgegebene Strahlung von mindestens 30 %, insbesondere mindestens 50 %, auf. Dadurch wird erreicht, dass über den Sensorkopf ausgegebene und auf den zweiten Bereich der Auflage des Positionierelements auftreffende elektromagnetischen Strahlung zum großen Teil reflektiert und wieder über den Sensorkopf in das Interferometer zurückgeführt wird. Entsprechend hoch sind die Intensitätswerte im entsprechenden Bildbereich der hierbei erhaltenen OCT-Bilder gegenüber Intensitätswerten in Bildbereichen, in denen das Reflexionsverhalten des zu untersuchenden Objekt wiedergegeben wird. Ein Übergang zwischen dem ersten und zweiten Bereich der Auflage ist dadurch in den OCT-Bildern mit sehr hoher Zuverlässigkeit erkennbar, so dass die Positionierung des Sensorkopfes bei einfacher Handhabung mit hoher Präzision erfolgen kann.

Es ist bevorzugt, dass das System derart ausgebildet ist, dass Bilder aus unterschiedlichen Tiefen des Objekts anhand der in den unterschiedlichen Tiefen des Objekts reflektierten Strahlung erzeugt und wiedergegeben werden, wobei in den aus den unterschiedlichen Tiefen des Objekts erhaltenen und wiedergegebenen Bildern ein Übergang zwischen Objekt und Positionierelement, insbesondere zwischen dem ersten und zweiten Bereich des Positionierelements, erkennbar ist. Dies hat den besonderen Vorteil, dass der zur Positionierung herangezogene Übergang zwischen dem ersten und zweiten Bereich in den OCT-Bildern nicht nur gut erkennbar ist, wenn das System OCT-Bilder in einem Tiefenbereich erfasst, in welchem auch das Positionierelement liegt, sondern in den OCT-Bildern auch dann präzise erkannt werden kann, wenn die jeweils erhaltenen OCT-Bilder aus einem Tiefenbereich innerhalb des Objekts stammen, welcher unterhalb des Positionierelements liegt. Dadurch kann eine Positionierung des Sensorkopfes relativ zum Objekt auch dann noch erfolgen, wenn während eines sog. Tiefenscans bei der Untersuchung des Objekts die Notwendigkeit einer erneuten oder geänderten Positionierung erkannt wird, ohne dass zusätzliche Steuerungsmaßnahmen ergriffen werden müssten.

Vorzugsweise umfasst das System einen Detektor zur Erfassung der vom Objekt und/oder vom Positionierelement reflektierten und in das Interferometer zurückgeführten Strahlung, wobei die Steuereinrichtung zur Ansteuerung des Detektors derart ausgebildet ist, dass sich dieser bei der Erfassung der vom Positionierelement reflektierten und in das Interferometer zurückgeführten Strahlung in einem Sättigungsbetrieb befindet. Dadurch wird ein maximal möglicher Kontrast zwischen den OCT-Bildbereichen, die dem ersten und zweiten Bereich der Auflage entsprechen, erreicht, so dass der Übergang zwischen dem ersten und zweiten Bereich mit besonders hoher Zuverlässigkeit erkannt werden kann. Dies trifft besonders dann zu, wenn die Bildsignale vor deren Wiedergabe als OCT-Bilder demoduliert werden, wodurch lediglich veränderliche Bildsignalverläufe in den OCT-Bildern zu erkennen sind, während das konstante Sättigungssignal lediglich als schwarzer Bereich in den OCT-Bildern in Erscheinung tritt.

Es ist außerdem besonders bevorzugt, dass die Bilderzeugungseinrichtung zur Erzeugung von Bildern des Objekts bzw. Positionierelements mit einer Rate von mehr als einem Bild pro Sekunde, insbesondere mehr als fünf Bildern pro Sekunde, ausgebildet ist und/oder die Anzeigeeinrichtung zur Wiedergabe von Bildern des Objekts bzw. Positionierelements mit einer Rate von mehr als einem Bild pro Sekunde, insbesondere mehr als fünf Bildern pro Sekunde, ausgebildet ist. Bei diesen Erzeugungs- bzw. Wiedergaberaten kann von einer Erzeugung bzw. Wiedergabe von OCT-Bildern in Echtzeit gesprochen werden, so dass eine Bedienperson anhand von Echtzeit-OCT-Bildern - d.h. anhand eines jeweils aktuellen OCT-Bildes - erkennen kann, ob der Sensorkopf die gewünschte Position relativ zum Objekt hat und sich insbesondere auf oder über dem ersten Bereich der Auflage des Positionierelements befindet, oder seine gegebenenfalls etwas verändert werden muss. Eine Positionsänderung des Sensorkopfes kann anhand der nachfolgenden und in Echtzeit aufgenommenen bzw. wiedergegebenen OCT-Bilder von der Bedienperson unmittelbar nach der Positionsänderung überprüft werden.

Bei dem zu untersuchenden Objekt handelt es sich vorzugsweise um biologisches Gewebe, insbesondere um das Hautorgan eines Menschen oder Tieres. Grundsätzlich kann die Erfindung aber auch zur Untersuchung anderer menschlicher oder tierischer Organe eingesetzt werden.

Weitere Vorteile, Merkmale und Anwendungsmöglichkeiten der vorliegenden Erfindung ergeben sich aus der nachfolgenden Beschreibung in Zusammenhang mit den Figuren. Es zeigen:
- Fig. 1: eine schematische Darstellung eines Beispiels eines Systems zur optischen Kohärenztomographie;
- Fig. 2: eine schematische Darstellung eines Beispiels einer Detektorfläche zur Veranschaulichung eines ersten Betriebsmodus;
- Fig. 3: ein Raumelement des Objekts mit Schnitten in ersten Ebenen zur Veranschaulichung des ersten Betriebsmodus;
- Fig. 4: ein Raumelement des Objekts mit einem Schnitt in einer zweiten Ebene zur Veranschaulichung eines zweiten Betriebsmodus;
- Fig. 5: ein Raumelement des Objekts mit Schnitten in zweiten Ebenen zur Veranschaulichung eines dritten Betriebsmodus;
- Fig. 6: a) und b) zwei Querschnitte durch das Objekt und den Probenarm des Interferometers zur Veranschaulichung der Fokusnachführung;
- Fig. 7: ein Beispiel für ein regelmäßiges Gitter zur Veranschaulichung der Interpolation von Ausgangsbildwerten;
- Fig. 8: ein Schema zur Veranschaulichung einer Abtastung eines Interferenzmusters in Richtung der Tiefe eines Objekts im Vergleich zur physikalischen Auflösung in Richtung der Tiefe;
- Fig. 9: ein weiteres Schema zur Veranschaulichung einer Zusammenfassung von in Richtung der Tiefe eines Objekts abgetasteten ursprünglichen Ausgangsbildwerten zu jeweils einem Ausgangsbildwert im Vergleich zur physikalischen Auflösung in Richtung der Tiefe;
- Fig. 10: ein weiteres Schema zur Veranschaulichung der Interpolation der Ausgangsbildwerte von zwei in Richtung der Tiefe des Objekts erhaltenen Ausgangsbildern;
- Fig. 11: ein weiteres Schema zur Veranschaulichung der Erfassung Ausgangsbildwerte in einer (links) bzw. zwei (rechts) Ebenen quer zur Richtung der Tiefe eines Objekts sowie der Interpolation der Ausgangsbildwerte der von den beiden Ebenen erhaltenen Ausgangsbildern (rechts);
- Fig. 12: ein Beispiel eines Ausgangsbildes (links) im Vergleich zu einem entsprechenden Endbild (rechts), welches durch die beschriebene Interpolation erhalten wurde;
- Fig. 13: ein weiteres Beispiel eines Systems zur optischen Kohärenztomographie;
- Fig. 14: eine Darstellung eines Sensorkopfes des Systems;
- Fig. 15: eine Darstellung des Sensorkopfes zusammen mit einem Positionierelement;
- Fig. 16: ein erstes Bespiel eines Positionierelements in Draufsicht auf die dem Sensorkopf zugewandten Seite (links) und die dem Objekt zugewandten Seite (rechts);
- Fig. 17: ein weiteres Bespiel eines Positionierelements in Draufsicht auf die dem Sensorkopf zugewandten Seite (links) und die dem Objekt zugewandten Seite (rechts);
- Fig. 18: eine Querschnittsdarstellung zur Veranschaulichung des Positioniervorgangs in unterschiedlichen Phasen a) bis c);
- Fig. 19: ein erstes Beispiel für OCT-Bilder, die mit einem opaken Positionierelement aufgenommen wurden (a) Slice, b) En-face);
- Fig. 20: ein zweites Beispiel für OCT-Bilder, die mit einem opaken Positionierelement aufgenommen wurden (a) Slice, b) En-face);
- Fig. 21: ein erstes Beispiel für OCT-Bilder, die mit einem stark reflektierenden Positionierelement aufgenommen wurden (a) Slice, b) En-face);
- Fig. 22: ein zweites Beispiel für OCT-Bilder, die mit einem stark reflektierenden Positionierelement aufgenommen wurden (a) Slice, b) En-face); und
- Fig. 23: ein drittes Beispiel für OCT-Bilder, die mit einem stark reflektierenden Positionierelement aufgenommen wurden (a) Slice, b) En-face);

### 1. Optische Kohärenztomographieeinrichtung

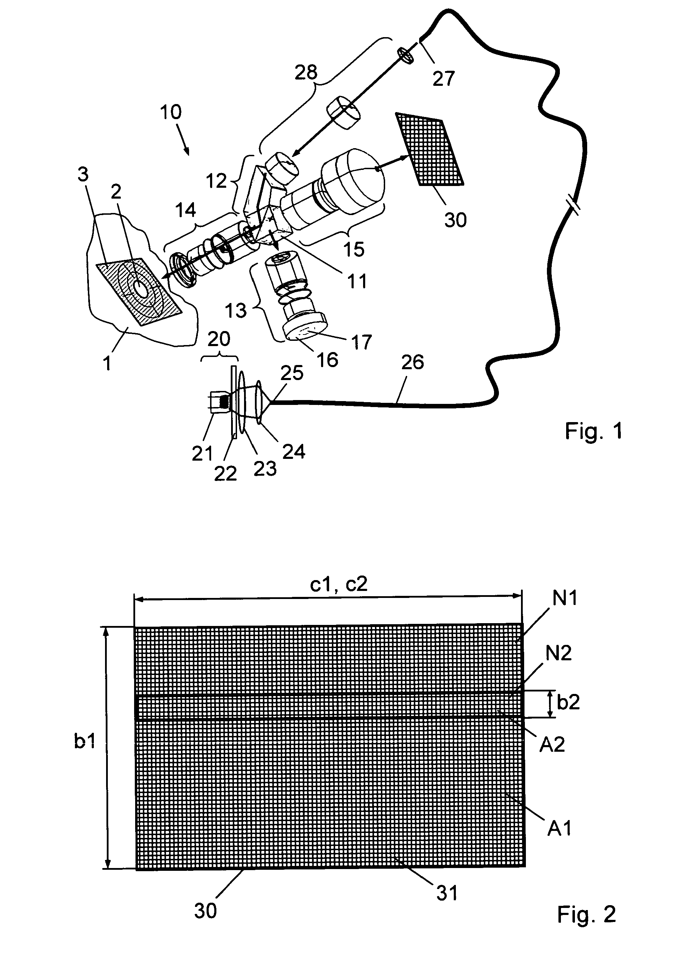

Figur 1 zeigt eine schematische Darstellung eines Beispiels für ein System zur optischen Kohärenztomographie, welches eine optische Kohärenztomographieeinrichtung, nachfolgend auch als OCT-Einrichtung bezeichnet, aufweist.

Die OCT-Einrichtung weist ein Interferometer 10 auf, welches einen Strahlteiler 11, einen Beleuchtungsarm 12, einen Referenzarm 13, einen Probenarm 14 sowie einen Detektorarm 15 umfasst. Ferner ist eine Strahlungsquelle 21 zur Erzeugung von Licht vorgesehen, welches durch einen optischen Filter 22 gefiltert wird und durch eine aus Linsen 23 und 24 zusammengesetzte Optik auf einen Eingangsbereich 25 eines Lichtleiters 26 fokussiert wird. Die Strahlungsquelle 21 bildet zusammen mit dem optischen Filter 22 eine Einrichtung, welche auch als Lichtquelle 20 bezeichnet wird.

Das in den Lichtleiter 26 eingekoppelte Licht wird durch eine in dessen Ausgangsbereich 27 befindliche Optik 28 in den Beleuchtungsarm 12 des Interferometers 10 eingekoppelt. Von dort gelangt das Licht zunächst zum Strahlteiler 11, durch welchen dieses einerseits in den Referenzarm 13 weitergeleitet und von einem an dessen Ende befindlichen beweglichen Referenzspiegel 16 reflektiert wird und andererseits nach Durchlaufen des Probenarms 14 eine Fläche 2 einer Probe 1 beleuchtet. Die Probe 1, bei welcher es sich insbesondere um biologisches Gewebe, insbesondere des menschlichen Hautorgans, handelt, wird im Zusammenhang mit der vorliegenden Erfindung auch als Objekt bezeichnet.

Um auf einfache Weise eine möglichst genaue Positionierung der OCT-Einrichtung, insbesondere des Probenarms 14 des Interferometers 10, auf der Probe 1 zu ermöglichen, ist ein Positionierelement 3 vorgesehen, das auf die Probe 1 aufgeklebt und nach der Bildaufnahme wieder von der Probe 1 entfernt werden kann. Das Positionierelement 3 weist zwei Bereiche mit jeweils unterschiedlicher Durchlässigkeit und/oder Reflexivität für das vom Interferometer 10 ausgegebene und/oder von der Probe 1 reflektierte Licht auf. Aufgrund der unterschiedlichen optischen Eigenschaften der beiden Bereiche können diese in den aufgenommenen und wiedergegebenen OCT-Bildern leicht unterschieden werden, so dass eine Bedienperson auf einfache Weise erkennen kann, ob die OCT-Einrichtung wie gewünscht zur Probe positioniert ist oder gegebenenfalls eine Positionskorrektur vorgenommen werden muss. Auf weitere Eigenschaften des Positionierelements 3 sowie Einzelheiten bei der mit Hilfe des Positionierelements 3 durchgeführten Positionierung eines Sensorkopfes wird weiter unten noch ausführlich eingegangen.

Das von der Probe 1 reflektierte, insbesondere zurückgestreute, Licht durchläuft erneut den Probenarm 14, wird im Strahlteiler 11 mit dem am Referenzspiegel 16 reflektierten Licht aus dem Referenzarm 13 überlagert und gelangt schließlich über den Detektorarm 15 auf einen Detektor 30, der eine Vielzahl von in einer, vorzugsweise ebenen, Fläche angeordneten Detektorelemente umfasst und folglich eine ortsaufgelöste Erfassung des von der Probe 30 reflektierten Lichts bzw. eines entsprechenden Interferenzmusters aufgrund dessen Überlagerung mit dem am Referenzspiegel 16 reflektierten Licht ermöglicht.

Als Detektor 30 wird vorzugsweise eine CMOS-Kamera eingesetzt, deren Detektorelemente (sogenannte Pixel) im infraroten Spektralbereich empfindlich sind, insbesondere in einem Spektralbereich zwischen etwa 1250 nm und 1350 nm. Vorzugsweise hat die CMOS-Kamera 512 x 640 Detektorelemente.

Als Lichtleiter 26 dient vorzugsweise eine sogenannte Multimode-Faser, deren numerische Apertur und Kerndurchmesser es zulassen, dass sich bei einer bestimmten Wellenlänge des in die Faser eingekoppelten Lichts nicht nur eine Fasermode ausbilden kann, sondern viele verschiedene Fasermoden angeregt werden können. Vorzugsweise beträgt der Durchmesser der verwendeten Multimode-Faser zwischen etwa 1 mm und 3 mm, insbesondere etwa 1,5 mm.

Die Größe der beleuchteten Fläche 2 auf der Probe 1 entspricht in etwa der Größe der beleuchteten Fläche 17 auf dem Referenzspiegel 16 und wird einerseits durch die am Eingangsbereich des Lichtleiters 26 befindliche Optik, welche im dargestellten Beispiel die Linsen 23 und 24 umfasst, und andererseits durch die im Ausgangsbereich des Lichtleiters 26 vorgesehene Optik 28 bestimmt.

Bei der beschriebenen OCT-Einrichtung wird das entstehende Interferenzmuster mit dem Detektor 30 erfasst, wobei ein entsprechendes Interferenzsignal erzeugt wird. Die Abtastrate des Detektors 30 zum Abtasten des Interferenzsignals muss dabei so gewählt werden, dass die zeitliche Variation des Interferenzmusters mit ausreichender Genauigkeit erfasst werden kann. Dies erfordert im Allgemeinen hohe Abtastraten, wenn hohe Geschwindigkeiten für einen Tiefenscan erzielt werden sollen.

Ein Tiefenscan wird bei dem beschriebenen System vorzugsweise dadurch realisiert, dass der optische Abstand des Referenzspiegels 16 vom Strahlteiler 11 während der Erfassung des von der Probe 1 reflektierten Lichts mit dem Detektor 30 mit einer Geschwindigkeit v um einen optischen Weg verändert wird, welcher wesentlich größer ist als die mittlere Wellenlänge des in das Interferometer 10 eingekoppelten Lichts. Vorzugsweise wird hierbei das in mindestens 100 unterschiedlichen Tiefen der Probe 1 reflektierte Licht vom Detektor 30 erfasst. Es ist insbesondere bevorzugt, dass der optische Weg periodisch mit einer Amplitude verändert wird, die wesentlich größer ist als die mittlere Wellenlänge des in das Interferometer 10 eingekoppelten Lichts. Die Änderung des optischen Abstands des Referenzspiegels 16 um dem optischen Weg bzw. mit der Amplitude ist vorzugsweise mindestens 100 mal, insbesondere mindestens 1000 mal, größer ist als die mittlere Wellenlänge des in das Interferometer 10 eingekoppelten Lichts. Aufgrund der großen Wege bei dieser Abstandsvariation wird diese Bewegung des Referenzspiegels 16 auch als makroskopische Bewegung bezeichnet.

Da die einzelnen Perioden eines Interferenzmusters im Allgemeinen jeweils zu mehreren Zeitpunkten abgetastet werden müssen, ist die maximal mögliche Scan-Geschwindigkeit in Richtung der Tiefe der Probe 1 abhängig von der maximal möglichen Abtastrate des Detektors 30. Bei Verwendung von schnellen Detektor-Arrays mit hoher räumlicher Auflösung, d.h. großer Anzahl von Detektorelementen pro Längeneinheit, liegt die maximale Abtastrate typischerweise im Bereich von etwa 1 kHz. Dies führt bei einer mittleren Wellenlänge des in das Interferometer eingekoppelten Lichts von beispielsweise 1300 nm zu einer maximalen Geschwindigkeit für den Tiefenscan von etwa 0,1 mm/s, wenn vier Punkte pro Periode einer Interferenzstruktur aufgenommen werden.

Zur Erhöhung der Geschwindigkeit des Tiefenscans wird in der vorliegenden OCT-Einrichtung der zeitliche Verlauf der Empfindlichkeit des Detektors 30 für das zu erfassende Licht mit einer Frequenz moduliert, die um bis zu 40 % größer oder kleiner sein kann als die Dopplerfrequenz f_{D}, wobei die Dopplerfrequenz f_{D} durch die mittlere Wellenlänge λ₀ des in das Interferometer 10 eingekoppelten Lichts und die Geschwindigkeit v des beweglichen Referenzspiegels 16 wie folgt gegeben ist: f_{D} = 2v/λ₀ Typische Frequenzen dieser Modulation liegen im Bereich zwischen 1 kHz und 25 kHz. Es ist besonders bevorzugt, dass die Frequenz der Modulation der Detektorempfindlichkeit ungleich der Dopplerfrequenz f_{D} ist.

Das von der Probe 1 reflektierte und auf den Detektor 30 treffende Licht überlagert sich hierbei mit der modulierten Empfindlichkeit des Detektors 30, so dass der Detektor 30 bei der Erfassung des auf den Detektor 30 treffenden Interferenzmusters anstelle eines hochfrequenten Interferenzsignals mit einer Vielzahl von Perioden ein niederfrequentes Schwebungssignal erzeugt, welches deutlich weniger Perioden aufweist als das hochfrequente Interferenzsignal. Bei der Abtastung dieser Schwebung sind daher deutlich weniger Abtastzeitpunkte pro Zeiteinheit erforderlich, ohne hierbei relevante Information zu verlieren, als bei einer Abtastung des hochfrequenten Interferenzsignals ohne die Modulation der Empfindlichkeit des Detektors 30. Bei einer gegebenen maximalen Abtastrate des Detektors 30 hat dies zur Folge, dass die maximale Geschwindigkeit für einen Tiefenscan des Systems um ein Vielfaches erhöht werden kann.

Die Empfindlichkeit des Detektors 30 lässt sich z.B. direkt oder mit einem vor dem Detektor 30 angeordneten steuerbaren elektronischen Shutter modulieren. Alternativ oder zusätzlich können Eigenschaften eines optischen Elements vor dem Detektor 30, wie z.B. die Durchlässigkeit eines Detektorobjektivs für das von der Probe 1 reflektierte Licht, moduliert werden. Im Vergleich zu Systemen mit einer konstanten Detektorempfindlichkeit wird hierdurch die Scan-Geschwindigkeit um den Faktor 4 oder sogar 8 erhöht.

Die Geschwindigkeit der Bewegung des Referenzspiegels 16 steht vorzugsweise in einer festen Beziehung zur Frequenz der Modulation der Empfindlichkeit des Detektors 30 und ist insbesondere so gewählt, dass in eine Periodendauer des entstehenden Schwebungssignals eine ganzzahlige Anzahl von Abtastzeitpunkten, vorzugsweise vier Abtastzeitpunkte, passen.

Die auf diese Weise abgetasteten Schwebungssignale müssen vor einer Visualisierung noch verarbeitet werden, da in diesen Signalen noch die Interferenzinformation enthalten ist. Die wesentliche Information, die visualisiert werden soll, ist die Amplitude und Tiefenposition der jeweiligen Interferenz, nicht jedoch die Interferenzstruktur selbst. Dazu muss das Schwebungssignal demoduliert werden, indem z.B. durch Fourier- oder Hilpert-Transformation die sog. Einhüllende des Schwebungssignals bestimmt wird.

Da die Phase des Schwebungssignals im Allgemeinen unbekannt ist und diese sich auch für unterschiedliche Schwebungssignale aus unterschiedlichen Tiefen unterscheiden kann, wird ein digitaler Demodulationsalgorithmus eingesetzt, der unabhängig von der Phase ist. Vorzugsweise werden für die Abtastung des Interferenzsignals mit vier Abtastzeitpunkten pro Periode sogenannte 90° Phase Shift-Algorithmen verwendet. Hierdurch wird eine schnelle Demodulation des Schwebungssignals erreicht.

Vorzugsweise umfasst eine Periode der Modulation der Empfindlichkeit des Detektors 30 zwei Teilperioden, wobei während einer ersten Teilperiode der Detektor empfindlich und während einer zweiten Teilperiode der Detektor unempfindlich ist für das zu erfassende Licht. Im Allgemeinen sind die erste und die zweite Teilperiode gleich lang. Es kann jedoch von Vorteil sein, die Dauer der ersten und zweiten Teilperiode unterschiedlich lang zu gestalten. Dies gilt beispielsweise dann, wenn die Intensität des von der Lichtquelle 20 abgegebenen bzw. in das Interferometer 10 eingekoppelten Lichts und/oder des von der Probe 1 reflektierten Lichts relativ gering ist. In diesen Fällen kann die erste Teilperiode so gewählt werden, dass deren Dauer länger ist als die Dauer der zweiten Teilperiode. Auf diese Weise wird auch bei geringen Lichtintensitäten neben einer hohen Tiefenscan-Geschwindigkeit ein hohes Signal-Rausch-Verhältnis und damit eine hohe Bildqualität gewährleistet.

Alternativ zur Empfindlichkeit des Detektors 30 kann auch die Intensität des in das Interferometer 10 eingekoppelten Lichts zeitlich moduliert werden, wobei hinsichtlich der bevorzugten Ausführungen sowie der vorteilhaften Wirkungen die Erläuterungen zur oben beschriebenen Modulation der Detektorempfindlichkeit entsprechend gelten.

Die Strahlungsquelle 21 umfasst vorzugsweise einen wendelförmigen Draht, welcher von einer transparenten Umhüllung, vorzugsweise aus Glas, umgeben ist. Vorzugsweise ist die Strahlungsquelle 21 als Halogenglühlampe, insbesondere Wolfram-Halogen-Glühlampe, ausgebildet, wobei als Draht ein Wolframdraht verwendet wird und das Innere der Umhüllung mit Gas gefüllt ist, welches ein Halogen, beispielsweise Jod oder Brom, enthält. Durch Anlegen einer elektrischen Spannung wird der wendelförmige Draht zum Glühen gebracht, wodurch dieser räumlich inkohärentes Licht aussendet. Unter räumlich inkohärentem Licht im Sinne der vorliegenden Erfindung ist Licht zu verstehen, dessen räumliche Kohärenzlänge kleiner ist als 15 µm und insbesondere nur einige wenige Mikrometer, d.h. zwischen etwa 1 µm und 5 µm, beträgt.

Das von der Strahlungsquelle 21 erzeugte räumlich inkohärente Licht passiert den optischen Filter 22, welcher als Bandpassfilter ausgebildet ist und im Wesentlichen nur für Licht innerhalb einer vorgebbaren spektralen Bandbreite durchlässig ist. Der optische Filter 22 weist eine glockenförmige oder gaußförmige spektrale Filtercharakteristik auf, wobei nur diejenigen spektralen Lichtanteile des von der Strahlungsquelle 21 erzeugten Lichts den optischen Filter 22 passieren können, welche innerhalb der vorgegebenen Bandbreite um eine mittlere Wellenlänge der glocken- bzw. gaußförmigen spektralen Filtercharakteristik liegen.

Unter einer gaußförmigen spektralen Filtercharakteristik im Sinne der Erfindung ist zu verstehen, dass die Durchlässigkeit des optischen Filters 22 für Licht mit bestimmten Wellenlängen λ proportional ist zu exp[-[(λ - λ₀)/2·Δλ]²]. wobei λ₀ die Wellenlänge angibt, bei welcher der optische Filter 22 seine maximale Durchlässigkeit aufweist, und Δλ die Standardabweichung bezeichnet, welche mit der Halbwertsbreite FWHM des gaußförmigen Durchlässigkeitsverlaufs wie folgt zusammenhängt: FWHM ≈ 2,35 · Δλ.

Unter einer glockenförmigen spektralen Filtercharakteristik ist ein spektraler Verlauf der Durchlässigkeit des optischen Filters 22 zu verstehen, welcher durch einen gaußförmigen Verlauf angenähert werden kann und/oder nur soweit von einem gaußförmigen Verlauf abweicht, dass dessen Fourier-Transformierte einen im Wesentlichen gaußförmigen Verlauf mit keinen Nebenmaxima oder nur wenigen, sehr niedrigen Nebenmaxima aufweist, deren Höhe maximal 5 % des Maximums der Fourier-Transformierten beträgt.

Durch die Verwendung einer Strahlungsquelle 21, welche *a priori* räumlich inkohärentes Licht erzeugt, wird bei der Erfassung des von der Probe 1 reflektierten Lichts mittels des zweidimensionalen ortsauflösenden Detektors 30 das Auftreten von sog. Geisterbildern aufgrund kohärenten Übersprechens von Lichtstrahlen aus unterschiedlichen Orten innerhalb der untersuchten Probe 1 vermieden. Auf die bei einer Verwendung von räumlich kohärenten Strahlungsquellen üblicherweise erforderlichen zusätzlichen Einrichtungen zur Zerstörung der räumlichen Kohärenz kann dadurch verzichtet werden.

Darüber hinaus kann hierdurch auf thermische Strahlungsquellen, wie z.B. Glüh- oder Halogenlampen, zur Erzeugung inkohärenten Lichts zurückgegriffen werden, die deutlich leistungsstärker und kostengünstiger sind als die häufig eingesetzten Superlumineszenzdioden (SLDs).

Durch die optische Filterung mit einer gauß- oder glockenförmigen Filtercharakteristik wird das von der Strahlungsquelle 21 erzeugte Licht in zeitlich teilkohärentes Licht mit einer zeitlichen Kohärenzlänge von vorzugsweise mehr als etwa 6 µm umgewandelt. Dies ist bei der beschriebenen OCT-Einrichtung vom Typ eines sog. Time-Domain-OCT, bei welchem die Länge eines Referenzarms 13 im Interferometer 10 verändert und mittels eines, vorzugsweise zweidimensionalen, Detektors 30 kontinuierlich die Intensität der auftretenden Interferenz erfasst wird, besonders vorteilhaft, da durch die Filterung des Lichts mittels des durch den optischen Filter 22 realisierten Bandpasses einerseits eine hohe laterale Auflösung des von der Probe 1 erfassten Bildes erreicht und durch die gauß- oder glockenförmige spektrale Filtercharakteristik des optischen Filters 22 andererseits ein Auftreten von störenden Nebenmaxima bei der FourierTransformation des mit dem Detektor erfassten Interferenzmusters, welche das Auftreten weiterer Geisterbilder verursachen würden, vermieden wird.

Insgesamt werden mit der beschriebenen OCT-Einrichtung auf einfache Weise OCT-Bilder mit hoher Auflösung und Bildqualität erhalten.

Im gezeigten Beispiel ist der optische Filter 22 zwischen der Strahlungsquelle 21 und der aus den beiden Linsen 23 und 24 gebildeten eingangsseitigen Optik angeordnet. Grundsätzlich ist es aber auch möglich, den optischen Filter 22 zwischen den beiden Linsen 23 und 24 oder aber zwischen der Linse 24 und dem Eingangsbereich 25 des Lichtleiters 26 vorzusehen. Grundsätzlich ist eine Anordnung des optischen Filters 22 besonders vorteilhaft, wenn die auf den optischen Filter 22 treffenden Lichtstrahlen nur eine geringe Divergenz aufweisen oder insbesondere parallel zueinander verlaufen, da hierdurch einerseits Reflexionsverluste an den Grenzflächen des optischen Filters 22 reduziert und andererseits ein Strahlversatz aufgrund von Lichtbrechung vermindert wird. Im gezeigten Beispiel ist daher eine Anordnung des optischen Filters 22 zwischen den beiden Linsen 23 und 24 der Optik besonders bevorzugt.

Alternativ oder zusätzlich ist es aber auch möglich, den optischen Filter 22 direkt auf die Umhüllung der Strahlungsquelle 21 aufzubringen. Dies hat den Vorteil, dass auf ein zusätzliches Filterbauteil verzichtet werden kann.

Alternativ oder zusätzlich ist es aber auch möglich, den optischen Filter 22 zwischen dem Ausgangsbereich 27 des Lichtleiters 26 und dem Beleuchtungsarm 12 anzuordnen, beispielsweise vor oder zwischen den Linsen der zwischen dem Ausgangsbereich 27 des Lichtleiters 26 und dem Eingang des Beleuchtungsarms 12 befindlichen Optik 28.

Bei einer einfachen und besonders zuverlässigen Variante umfasst der optische Filter 22 einen Absorptionsfilter, insbesondere ein sogenanntes Masseglas, und einen Interferenzfilter, wobei auf das Masseglas mehrere, vorzugsweise zwischen etwa 30 und 70, dünne Schichten mit unterschiedlichen Brechungsindizes, beispielsweise durch Aufdampfen, aufgebracht werden, wodurch ein Interferenzfilter erhalten wird.

Für den Fall, dass der optische Filter 22 in die Umhüllung der Strahlungsquelle 21 integriert wird, wird der optische Filter 22 vorzugsweise durch Aufbringen solcher Interferenzschichten auf die Umhüllung realisiert. Alternativ oder zusätzlich ist es aber auch möglich, eine oder mehrere der Linsen 23, 24 bzw. der Linsen der Optik 28 mit einem entsprechenden Interferenzfilter zu versehen.

### 2. Betriebsmodi der OCT-Einrichtung

Die beschriebene OCT-Einrichtung kann in drei unterschiedlichen Betriebsmodi betrieben werden. Bei den Betriebsmodi handelt es sich um zwei Echtzeitmodi, in denen OCT-Bilder der Probe 1 mit einer hohen Rate von mindestens einem Bild pro Sekunde, vorzugsweise etwa 5 bis 10 Bildern pro Sekunde, erzeugt werden, sowie einen statischen Betriebsmodus.

Im ersten Betriebsmodus, dem Echtzeitmodus 1, werden in Echtzeit zweidimensionale Tiefenschnitte der Probe 1 erzeugt (sog. Slices). Dies wird dadurch realisiert, dass als Detektor 30 eine CMOS-Kamera verwendet wird, welche die Einstellung eines sog. Window of Interest (WOI) zulässt, bei welcher lediglich eine Teilfläche des Detektors 30 für Licht sensitiv ist und dieses in entsprechende Detektorsignale umwandelt. Die Reduzierung der sensitiven Kamerafläche ist verbunden mit einer deutlichen Erhöhung der Kamerageschwindigkeit, so dass bei dieser Einstellung mehr Kamerabilder pro Sekunde erzeugt werden können als im Vollbild-Modus.

Im Echtzeitmodus 1 wird vorzugsweise ein WOI gewählt, das in einer Richtung der gesamten Kameralänge bzw. -breite entspricht (z.B. 640 Pixel) und in der anderen Richtung die - durch den Typ der jeweiligen Kamera gegebene - minimal mögliche Anzahl von Pixel aufweist (z.B. 4 Pixel). Dadurch wird die Geschwindigkeit der Kamera so weit erhöht, dass OCT-Bilder in Echtzeit aufgenommen werden können.

Dies wird vorzugsweise in Kombination mit der oben beschriebenen Modulation der Empfindlichkeit des Detektors 30 bzw. der Modulation der Intensität des in das Interferometer 10 eingekoppelten Lichts bzw. vom Interferometer 10 ausgegebenen Lichts erreicht.

Figur 2 zeigt als Beispiel einen Detektor 30 mit einer Detektorfläche A1, welche eine erste Anzahl N1 von in einer Ebene angeordneten Detektorelementen 31 umfasst und eine Länge c1 und eine Breite b1 aufweist. Bei der o.g. Einstellung eines WOI wird Licht lediglich von den in einer Teilfläche A2 der Detektorfläche A1 befindlichen Detektorelementen 31 erfasst und in entsprechende Detektorsignale umgewandelt. Die zweite Anzahl N2 der Detektorelemente 31 der Teilfläche A2 ist kleiner als die erste Anzahl N1 der Detektorelemente 31 der gesamten Detektorfläche A1. Die Längen c1 und c2 der Detektorfläche A1 bzw. Teilfläche A2 sind gleich groß, während die Breiten b1 und b2 der Detektorfläche A1 bzw. Teilfläche A2 unterschiedlich sind.

Im gezeigten Beispiel ist die Teilfläche A2 nur vier Pixel breit, wohingegen die Detektorfläche A1 512 Pixel breit ist. Die sensitive Fläche der Detektorfläche A1 wird also um einen Faktor von 128 reduziert, was die für die Erfassung von Interferenzmustern und deren Umwandlung in entsprechende Detektorsignale erforderliche Zeitdauer erheblich verkürzt.

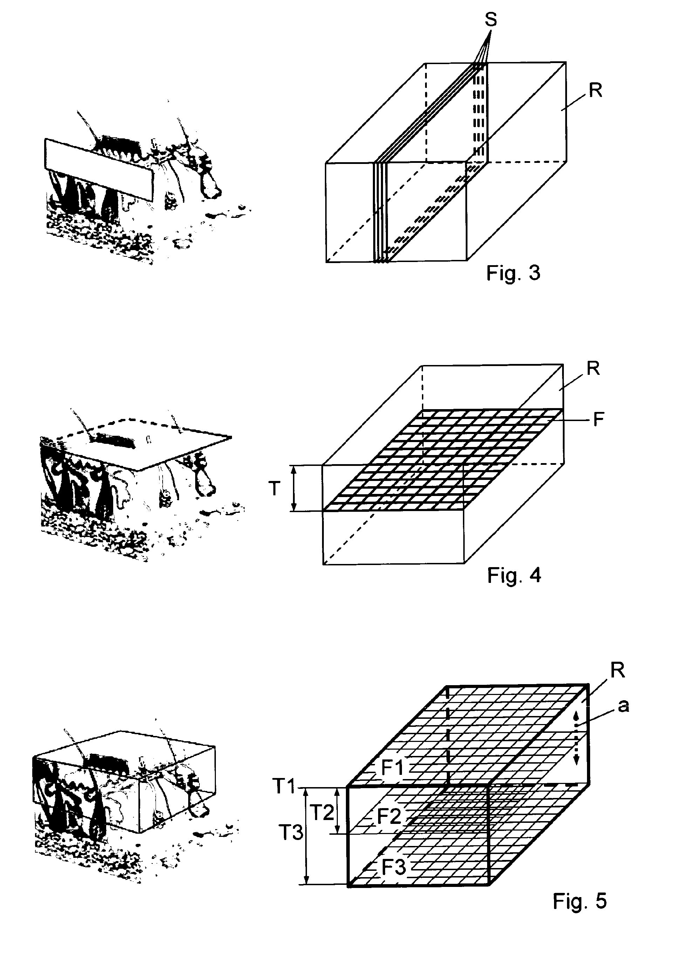

Wie in Figur 3 dargestellt ist, werden in diesem Beispiel anstelle eines vollen dreidimensionalen Tomogramms nur vier (entsprechend den vier Pixelreihen der Teilfläche A2) zweidimensionale Tiefenschnitte S (sogenannte Slices) aus dem betrachteten Raumelement R der Probe 1 erhalten. Aufgrund der im ersten Betriebsmodus erhaltenen Slices wird dieser Modus auch als Slice-Modus bezeichnet.

Zur weiteren Veranschaulichung zeigt der linke Teil der Figur 3 ein Modell der menschlichen Haut, in welches beispielhaft eine Ebene eines im Betriebsmodus 1, vorzugsweise in Echtzeit, aufgenommenen zweidimensionalen Tiefenschnittes bzw. Slices eingezeichnet ist.

Im zweiten Betriebsmodus, dem Echtzeitmodus 2, werden - wie in Figur 4 dargestellt - zweidimensionale Tomogramme F aus einer bestimmten Tiefe T des betrachteten Raumelements R der Probe 1 erzeugt, wobei die Tiefe T frei wählbar ist. Hierbei wird die gesamte Detektorfläche A1 des Detektors 30 für die Erfassung des von der Probe 1 reflektierten Lichts und dessen Umwandlung in entsprechende Detektorsignale genutzt, wobei jedoch nur jeweils maximal fünf Kamerabilder zur Berechnung eines Tomogramms F herangezogen werden. Dazu wird der Referenzspiegel 16 im Interferometer 10 bei einem bestimmten Abstand zum Strahlteiler 11 mit einer Amplitude von etwa 1 µm periodisch um diesen Abstand herum bewegt, während bis zu fünf Kamerabilder aufgenommen werden, die dann zu einem OCT-Bild verrechnet werden. Auf diese Weise können Tomogramme F mit hoher Wiederholungsrate, insbesondere in Echtzeit, erzeugt werden. Im Verhältnis zur oben beschriebenen makroskopischen Bewegung des Referenzspiegels 16 handelt es sich hierbei um eine mikroskopische Bewegung des Referenzspiegels 16.

Durch die makroskopische Bewegung des Referenzspiegels 16 - ggf. in Kombination mit einer weiter unten näher beschriebenen Fokusnachführung des in einer bestimmten Tiefe T in der Probe mittels der im Probenarm 14 befindlichen Probenoptik fokussierten Lichts - kann die Tiefe T, aus der das Tomogramm F gewonnen wird, frei gewählt werden.

Aufgrund der im zweiten Betriebmodus erhaltenen zweidimensionalen Schnitte F durch die Probe 1 in Ebenen, die im Wesentlichen senkrecht zur Richtung des auf die Probe 1 treffenden Lichts verlaufen, wird der zweite Betriebsmodus auch als En-face-Modus bezeichnet.

Zur weiteren Veranschaulichung zeigt der linke Teil der Figur 4 ein Modell der menschlichen Haut, in welches beispielhaft eine Ebene eines im Betriebsmodus 2, vorzugsweise in Echtzeit, aufgenommenen zweidimensionalen Tomogramms bzw. En-face-Bildes eingezeichnet ist.

Im dritten Betriebsmodus, einem statischen Modus, wird ein vollständiger dreidimensionaler Datensatz mit Hilfe der makroskopischen Bewegung des Referenzspiegels 16 in Kombination mit einer Fokusnachführung aufgenommen.

Bei einer mittleren Wellenlänge des in das Interferometer 10 eingekoppelten Lichts im Bereich von beispielsweise 1 µm liegt die optische Weglänge bzw. Amplitude der makroskopischen Bewegung des Referenzspiegels 16 mindestens bei etwa 0,1 mm, vorzugsweise mindestens bei etwa 1 mm.

Im Gegensatz zu der üblichen mikroskopischen Amplitude der Referenzspiegelbewegung in der Größenordnung von Bruchteilen der mittleren Wellenlänge des eingekoppelten Lichts, also von bis zu typischerweise 1 µm, erfolgt bei der beschriebenen OCT-Einrichtung eine makroskopische Bewegung des Referenzspiegels 16 in der Größenordnung von 0,1 mm bis zu mehreren Millimetern.

Während der makroskopischen Linearbewegung des Referenzspiegels 16 wird das von der Probe 1 reflektierte Licht über das Interferometer 10 zum zweidimensionalen Detektor 30 weitergeleitet und von diesem sukzessive zu mehreren Zeitpunkten für jeweils eine bestimmte Zeitdauer, welche der Integrationszeit des Detektors 30 entspricht, erfasst und in entsprechende Detektorsignale umgewandelt.

Damit eine Interferenz zwischen dem vom Referenzspiegel 16 und dem von der Probe 1 reflektierten Licht auftreten kann, muss die sog. Kohärenzbedingung erfüllt sein, welche u.a. besagt, dass die jeweils reflektierten Lichtwellen eine konstante Phasenbeziehung zueinander haben müssen, um miteinander interferieren zu können. Aufgrund der Verwendung von Licht mit einer sehr kurzen Kohärenzlänge von typischerweise 10 µm oder weniger ist die Bedingung einer konstanten Phasenbeziehung nur in bestimmten Tiefen oder Tiefenbereichen der Probe 1 erfüllt, welche auch als Kohärenz-Gate bezeichnet werden.

Jede Position des Referenzspiegels 16 während der makroskopischen Bewegung entspricht dabei einer bestimmten Tiefe innerhalb der Probe 1 oder einem Tiefenbereich um diese bestimmte Tiefe herum, für welche bzw. welchen die Kohärenzbedingung erfüllt ist, so dass eine Interferenz zwischen dem vom Referenzspiegel 16 und dem von der Probe 1 reflektierten Licht auftreten kann.

Im Falle einer periodischen Bewegung des Referenzspiegels 16 können beide Halbperioden der periodischen Bewegung des Referenzspiegels 16 jeweils zur Aufnahme von Detektorsignalen genutzt werden.

Auf diese Weise werden durch den Detektor 30 sukzessive zweidimensionale Schnitte aus unterschiedlichen Tiefen der Probe 1 aufgenommen. Dies ist in Figur 5 veranschaulicht, in welcher - stellvertretend für eine Vielzahl von zweidimensionalen Schnitten - ein erster, zweiter und dritter zweidimensionaler Schnitt F1, F2 bzw. F3 durch ein Raumelement R der Probe 1 dargestellt ist. Ein solcher zweidimensionaler Schnitt "wandert" synchron mit der makroskopischen Bewegung des Referenzspiegels 16 in Richtung a durch das betrachtete Raumelement R der Probe 1, ohne dass diese selbst bewegt werden muss.

Jeder Schnitt F1, F2 bzw. F3 liegt in einer Tiefe T1, T2 bzw. T3 der Probe 1, in welcher jeweils die Kohärenzbedingung erfüllt ist, so dass eine Interferenz zwischen dem vom Referenzspiegel 16 und dem von der Probe 1 reflektierten Licht auftreten kann. Die makroskopische Bewegung des Referenzspiegels 16 in Kombination mit der sukzessiven zweidimensionalen Erfassung des von der Probe 1 reflektierten Lichts hat somit die Wirkung eines dreidimensionalen Tiefenscans.

Die oben beschriebene Kombination der makroskopischen Linearbewegung des Referenzspiegels 16 einerseits mit der Erfassung des von der Probe 1 reflektierten Lichts mit einem zweidimensionalen Detektor 30 andererseits ermöglicht eine einfach zu realisierende und schnelle Aufnahme eines vollständigen dreidimensionalen Datensatzes des gewünschten Raumelements R der Probe 1.

Durch die makroskopische Bewegung des Referenzspiegels 16 wird hierbei ein dreidimensionales Tomogramm anstatt eines nur zweidimensionalen Bildes aus einer bestimmten Tiefe erhalten. Dabei braucht die Probe 1 zur Aufnahme eines dreidimensionalen Datensatzes relativ zum zweiten Interferometer 20 nicht mehr bewegt zu werden. Dies macht die beschriebene OCT-Einrichtung kompakt, zuverlässig und einfach handhabbar, so dass dieses besonders für den Einsatz in vivo geeignet ist.

Zur weiteren Veranschaulichung zeigt der linke Teil der Figur 5 ein Modell der menschlichen Haut, in welches beispielhaft ein Raumelement eingezeichnet ist, von welchem im Betriebsmodus 3 ein dreidimensionales Tomogramm aufgenommen wird.

### 3. Fokusnachführung

Die oben beschriebene OCT-Einrichtung ist so entworfen, dass während eines vollen Hubs, d.h. der Weglänge bzw. der doppelten Amplitude, der Bewegung des Referenzspiegels 16 stets ein Interferenzsignal mit ausreichend hoher Intensität und hoher Schärfe erhalten wird. Durch die nachfolgend näher beschriebene Fokusnachführung wird darüber hinaus gewährleistet, dass das Interferenzsignal sowie die Schärfe des erfassten Interferenzmusters für alle Tiefen in der Probe 1 maximal sind.

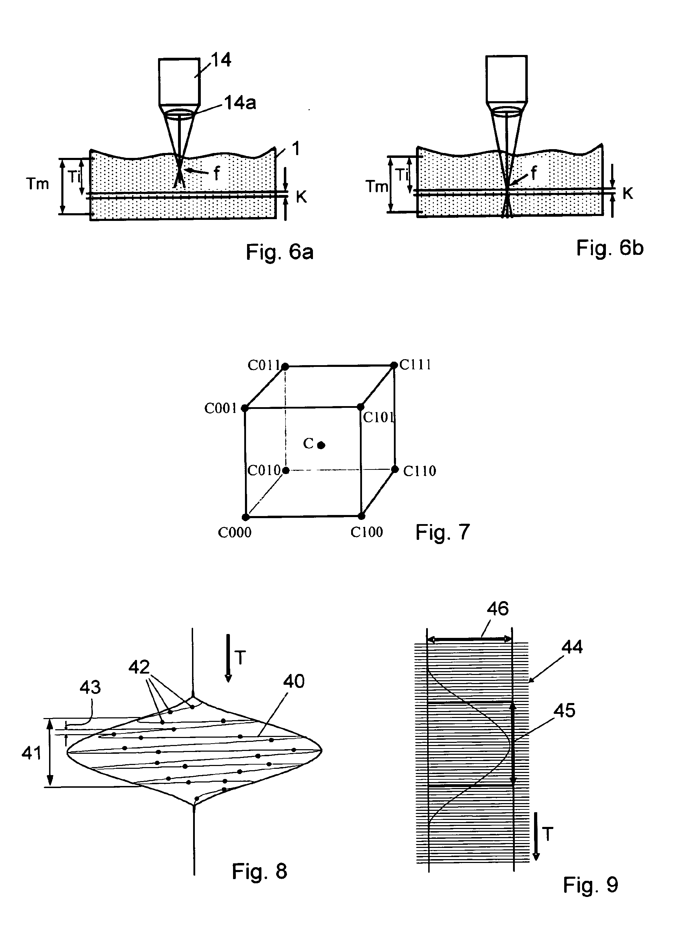

Dazu wird während der Erfassung des von der Probe 1 reflektierten Lichts der Fokus, d.h. der Brennpunkt, der im Probenarm 14 befindlichen Abbildungsoptik des Interferometers 10 in der Weise eingestellt, dass die Lage des Fokus in der Probe 1 und die Lage derjenigen Ebene in der Probe 1, bei welcher im Falle einer Reflexion von Licht die Kohärenzbedingung erfüllt ist und Interferenz auftritt, zu allen Zeiten während der Aufnahme eines Tomogramms des Raumelements R der Probe 1 im Wesentlichen identisch sind. Dies wird nachfolgend anhand der Figuren 6a und 6b veranschaulicht.

Figur 6a zeigt den Fall, bei welchem der Fokus f des - hier nur vereinfacht als Linse dargestellten - Probenobjektivs 14a im Probenarm 14 in einer Tiefe der Probe 1 liegt, die nicht mit der Lage des Kohärenz-Gates K übereinstimmt. Der innerhalb des Kohärenz-Gates K in der Tiefe Ti erfasste Schnitt durch die Probe 1 wird dadurch nicht exakt scharf auf den Detektor 30 (siehe Fig. 1) abgebildet, so dass Informationsverluste bei der Erfassung der Interferenz hinzunehmen wären.

In Figur 6b ist dagegen der Fall dargestellt, bei welchem der Fokus f des Probenobjektivs 14a derart eingestellt wurde, dass er innerhalb des Kohärenz-Gates K in der Tiefe Ti liegt. Dieses Nachführen des Fokus f des Probenobjektivs 14a entsprechend der jeweiligen Tiefe Ti des Kohärenz-Gates K wird als Fokus Tracking bezeichnet. Auf diese Weise wird das Interferometer 10 während des Tiefenscans auf die jeweilige Lage des Kohärenz-Gates K in unterschiedlichen Tiefen Ti der Probe 1 scharf gestellt, so dass aus jeder Tiefe der Probe 1 Bilder mit hoher Schärfe erhalten werden.

Die maximale optische Scantiefe Tm gibt an, bis zu welcher Tiefe unterhalb der Oberfläche der Probe 1 die Kohärenzbedingung für eine konstruktive Interferenz erfüllt und entsprechende Interferenzmuster erhalten werden.

Das in den Figuren 6a und 6b vereinfacht dargestellte Probenobjektiv 14a umfasst vorzugsweise mehrere Linsen, die einzeln und/oder gruppenweise in Richtung auf die Probe 1 bzw. von dieser weg bewegt werden können. Hierzu ist z.B. ein piezoelektrischer Aktuator, insbesondere ein Ultraschall-Piezo-Motor, vorgesehen, der mit dem Probenobjektiv 14a bzw. den Linsen gekoppelt ist und dieses bzw. diese entlang einer oder mehrer Führungen, insbesondere Führungsstäbe oder Führungsnuten, bewegt.

Die Bewegung des Probenobjektivs 14a bzw. der Linsen erfolgt vorzugsweise synchron mit der makroskopischen Bewegung des Referenzspiegels 16 im Interferometer 10 (siehe Fig. 1). Auf diese Weise folgt der Fokus f des Probenobjektivs 14a dem Kohärenz-Gate G, während Letzteres sukzessive unterschiedliche Tiefen T1, T2 bzw. T3 der Probe 1 durchfährt, aus denen mit Hilfe des Detektors 30 jeweils zweidimensionale Schnitte F1, F2 bzw. F3 (vgl. Fig. 5) aufgenommen werden.

Die Synchronisation der makroskopischen Bewegung des Referenzspiegels 16 und der Fokusnachführung einerseits in Kombination mit einem zweidimensionalen Detektor 30 andererseits gewährleistet eine besonders einfache und schnelle Aufnahme einer Vielzahl von scharfen zweidimensionalen Bildschnitten in unterschiedlichen Tiefen der Probe 1 und damit die Erfassung eines vollen dreidimensionalen Bilddatensatzes mit hoher Bildqualität.

Da das Interferometer 10 und die optische Abbildung im Probenarm 14 kontinuierlich aufeinander abgestimmt werden, sind die vom Detektor 30 erfassten Interferenzsignale für jede Tiefe in der Probe 1 maximal, so dass sich ein sehr hohes Signal-zu-Rausch-Verhältnis ergibt. Darüber hinaus wird dadurch sichergestellt, dass die laterale Auflösung für alle Tiefen in der Probe 1 optimal ist, da der Fokus f der Abbildung stets im Kohärenz-Gate K liegt. Es werden dadurch detailgetreue OCT-Bilder mit hohem Kontrast erhalten.

Vorteilhafterweise ist die Geschwindigkeit v2 der Bewegung der Linse bzw. Linsen des Probenobjektivs 14a in Richtung der Probe 1 kleiner als die Geschwindigkeit v1 der Bewegung des Referenzspiegels 16. Vorzugsweise wird hierbei ein Verhältnis v1/v2 der Geschwindigkeiten des Referenzspiegels 16 und der Linsen gewählt, das näherungsweise gleich 2-n - 1 ist oder bis zu etwa ± 20 %, vorzugsweise bis zu etwa ± 10 %, um diesen Wert liegt. Hierdurch werden die Lage des Fokus f und Kohärenz-Gates G mit besonders hoher Zuverlässigkeit aufeinander abgestimmt.

Durch die oben beschriebene Wahl des Verhältnisses v1/v2 der Geschwindigkeiten des Referenzspiegels 12 und der Linsen 42 wird gewährleistet, dass das Kohärenz-Gate K und der Fokus f während des Tiefenscans im gesamten betrachteten Tiefenbereich übereinander liegen. Im obigen Beispiel einer Probe mit einem Brechungsindex n = 1,4 liegt das Verhältnis v1/v2 der Geschwindigkeiten im Bereich von etwa (2·1,4 - 1) ± 20 %, d.h. zwischen etwa 1,44 und 2,16, und beträgt vorzugsweise etwa 2·1,4 - 1 = 1,8.

### 4. Trilineare Interpolation

Die mit der oben beschriebenen OCT-Einrichtung bzw. Methode erhaltenen OCT-Bilder können zur weiteren Verbesserung der Erkennung diagnostischer Information, beispielsweise im Bereich der Dermatologie zur noch besseren Erkennung von Hohlräumen oder Verdickungen in der Haut mit einer Größe von mehr als etwa 10 µm, einer Interpolation unterzogen werden.

Eine bei den mit der oben beschriebenen OCT-Einrichtung bzw. Methode erhaltenen OCT-Bildern, insbesondere Echtzeitbildern, besonders vorteilhafte Interpolationsmethode stellt hierbei die sog. trilineare Interpolation dar, bei welcher die Ausgangsbildwerte von mindestens zwei zweidimensionalen Ausgangsbildern, die in parallel zueinander verlaufenden Ebenen des Objekts aufgenommen wurden, im dreidimensionalen Raum interpoliert werden, so dass ein zweidimensionales Endbild erhalten wird. Dies wird nachfolgend im Einzelnen näher erläutert.

Bei der trilinearen Interpolation handelt es sich um ein Verfahren zur multivarianten Interpolation in einem dreidimensionalen regelmäßigen Gitter, d.h. einem Gitter mit gleicher Gitterkonstante in allen drei Raumrichtungen. Dies wird anhand eines in Figur 7 beispielhaft gezeigten Gitters veranschaulicht. Aus einer Interpolation der an den acht Ecken C000 bis C111 eines Würfels befindlichen Ausgangsbildwerten wird jeweils ein im Mittelpunkt C des Würfels befindlicher Interpolationswert abgeleitet.

Die jeweiligen Ausgangsbildwerte stammen von in verschiedenen Ebenen des Objekts aufgenommenen Ausgangsbildern. Bei den Ausgangsbildwerten handelt es sich um Lichtintensitätswerte an unterschiedlichen Orten in den entsprechenden zweidimensionalen Ausgangsbildern. Beispielsweise stammen die Ausgangsbildwerte, d.h. die Lichtintensitätswerte, mit den Koordinaten C000, C001, C011 und C010 von einem im Betriebsmodus 1 aufgenommenen ersten Echtzeitbild entlang eines ersten Tiefenschnittes S (siehe Fig. 3) und die Ausgangsbildwerte, d.h. die Lichtintensitätswerte, mit den Koordinaten C100, C101, C111 und C110 von einem im Betriebsmodus 1 aufgenommenen zweiten Echtzeitbild entlang eines davon im Abstand der Gitterkonstante beabstandeten zweiten Tiefenschnittes S (siehe Fig. 3). In einem alternativen Beispiel stammen die Ausgangsbildwerte mit den Koordinaten C000, C010, C110 und C100 von einem im Betriebsmodus 2 aufgenommenen ersten Echtzeitbild in Form eines ersten zweidimensionalen Tomogramms F (siehe Fig. 4) und die Ausgangsbildwerte mit den Koordinaten C001, C011, C111 und C101 von einem im Betriebsmodus 2 aufgenommenen zweiten Echtzeitbild in Form eines im Abstand der Gitterkonstante davon beabstandeten zweiten zweidimensionalen Tomogramms F (siehe Fig. 4).

Für eine trilineare Interpolation der mit der oben beschriebenen OCT-Einrichtung bzw. Methode erhaltenen OCT-Bilder, insbesondere der Echtzeitbilder, wird eine identische Auflösung in allen drei Raumdimensionen gewählt.

Bei aus dem Stand der Technik bekannten OCT-Systemen ist dies nicht ohne Auflösungsverlust erreichbar, da meist nur eine relativ hohe axiale (d.h. longitudinale, in Richtung des auf das Objekt treffenden Lichts) Auflösung realisiert werden kann, wohingegen die laterale (d.h. transversale, senkrecht zur Richtung des auf das Objekt treffenden Lichts) Auflösung meist deutlich niedriger ist. Eine Wahl gleicher Auflösung in allen drei Raumrichtungen wäre daher nur durch eine Erniedrigung der axialen Auflösung möglich, was jedoch wegen des großen Informationsverlusts in der Regel nicht wünschenswert ist, da dann kleine Objekte nicht mehr aufgelöst werden könnten. Außerdem ist es bei aus dem Stand der Technik bekannten OCT-Systemen nicht möglich, zwei zweidimensionale Bilder gleichzeitig oder zumindest fast gleichzeitig aufzunehmen. Dies gilt insbesondere für En-face-Bilder und scannende Systeme. Eine trilineare Interpolation in Echtzeit ist dadurch nahezu unmöglich, da dann auch Bewegungsartefakte relevant werden.

Bei den mit der oben beschriebenen OCT-Einrichtung bzw. Methode erhaltenen OCT-Bildern ist dagegen eine trilineare Interpolation sowohl im Falle der in den Betriebsmodi 1 und 2 erfassten zweidimensionalen Echtzeitbildern (Slice bzw. En-face) als auch zur Nachbearbeitung der im statischen Betriebsmodus 3 erhaltenen dreidimensionalen Tomogrammen möglich.

Bei der oben beschriebenen OCT-Einrichtung wird die axiale (d.h. longitudinale) Auflösung im Wesentlichen durch die spektrale Bandbreite der Lichtquelle 20 und den Brechungsindex des zu untersuchenden Objekts 1 bestimmt, während die laterale (d.h. transversale) Auflösung im Wesentlichen durch die optische Abbildung und die Größe der Detektorelemente 31 des Detektors 30 (siehe Figuren 1 und 2) bestimmt wird.

Die oben beschriebene OCT-Einrichtung ist so abgestimmt, dass laterale und axiale Auflösung nahezu gleich und sehr hoch sind. Vorzugsweise liegt die Auflösung in allen drei Dimensionen bei ca. 3 µm x 3 µm x 3µm.

Dies wird für die laterale Auflösung insbesondere durch die oben beschriebene Fokusnachführung und für die axiale Auflösung insbesondere durch die Verwendung einer Lichtquelle 20 erreicht, die eine Halogenlampe als Strahlungsquelle 21 in Kombination mit einem Gauß-Filter 22 umfasst.

Es ist außerdem bevorzugt, dass die Schärfentiefe der abbildenden Optik, insbesondere des Probenobjektivs 14, des Interferometers 10 (siehe Figur 1) größer ist als der "Gitterabstand" der Ausgangsbildwerte, d.h. der räumliche Abstand der Ausgangsbildwerte in den drei Dimensionen. Dadurch wird in jedem Fall gewährleistet, dass die Ausgangsbildwerte stets mit hoher Genauigkeit erfasst werden.

Vorzugsweise wird darüber hinaus der Tatsache Rechnung getragen, dass die Abtastung des Interferenzsignals hoch genug sein muss, um das sog. Abtasttheorem nicht zu verletzen. Dies wird nachfolgend näher erläutert.

Figur 8 zeigt ein Schema zur Veranschaulichung einer Abtastung eines Interferenzmusters 40 in Richtung der Tiefe T eines Objekts im Vergleich zur physikalischen Auflösung 41 in Richtung der Tiefe T. Bei der oben beschriebenen OCT-Einrichtung bzw. Methode werden vorzugsweise jeweils vier Punkte 42 pro Interferenzperiode des Interferenzmusters 40 abgetastet. Eine Interferenzperiode ist hierbei eine halbe (mittlere) Wellenlänge des in das Interferometer eingekoppelten Lichts lang (bei einer mittleren Wellenlänge von ca. 1,3 µm entspricht dies ca. 0,65 µm). Daraus folgt, dass der Abstand 43 zweier Abtastpunkte 42 ca. 0,163 µm beträgt. Die physikalische Auflösung 41 in Luft beträgt jedoch etwa 4 µm. Dies bedeutet, dass etwa 24 aufeinander folgende Zeilen in Tiefenrichtung T annähernd dieselbe physikalische Information enthalten und daher ohne signifikanten Informationsverlust zu einer Zeile zusammengefasst werden können. Dies wiederum hat zur Folge, dass der resultierende Volumenbildpunkt (sog. Voxel) in allen drei Dimensionen nahezu gleiche Ausdehnung hat, also im Wesentlichen einem Würfel entspricht. Der Ausgangsbildwert entspricht dabei beispielsweise einem Mittelwert oder dem Median aus den ursprünglichen Ausgangsbildwerten.

Figur 9 veranschaulicht das oben beschriebene Zusammenfassen von in Richtung der Tiefe T des Objekts in mehreren aufeinanderfolgenden Zeilen 44 abgetasteten ursprünglichen Ausgangsbildwerten zu einer Zeile mit nur einem Ausgangsbildwert und einer Zeilenhöhe, d.h. einer longitudinalen Ausdehnung 45 in Tiefenrichtung T, die der lateralen Ausdehnung 46 eines Bildpunktes (Pixels) der Zeile senkrecht zur Tiefenrichtung T entspricht.

Im Betriebsmodus 1, bei welchen Slices in Echtzeit aufgenommen werden, werden bei der trilinearen Interpolation zwei benachbarte Zeilen des Detektors 30 gleichzeitig ausgelesen. Im Beispiel des in Figur 2 gezeigten Detektors 30 bedeutet dies, dass die Breite b2 der Teilfläche A2 des Detektors 30 so gewählt wird, dass sich diese in Richtung der Breite des Detektors 30 nur über zwei Detektorelemente 31 erstreckt. Die Teilfläche A2 umfasst dann nur 2 x 640 Detektorelemente 31, die während einer makroskopischen Bewegung des Referenzspiegels16 (siehe Figur 1) sukzessive ausgelesen und in der oben beschriebenen Weise zu einem zweidimensionalen Endbild verrechnet werden.

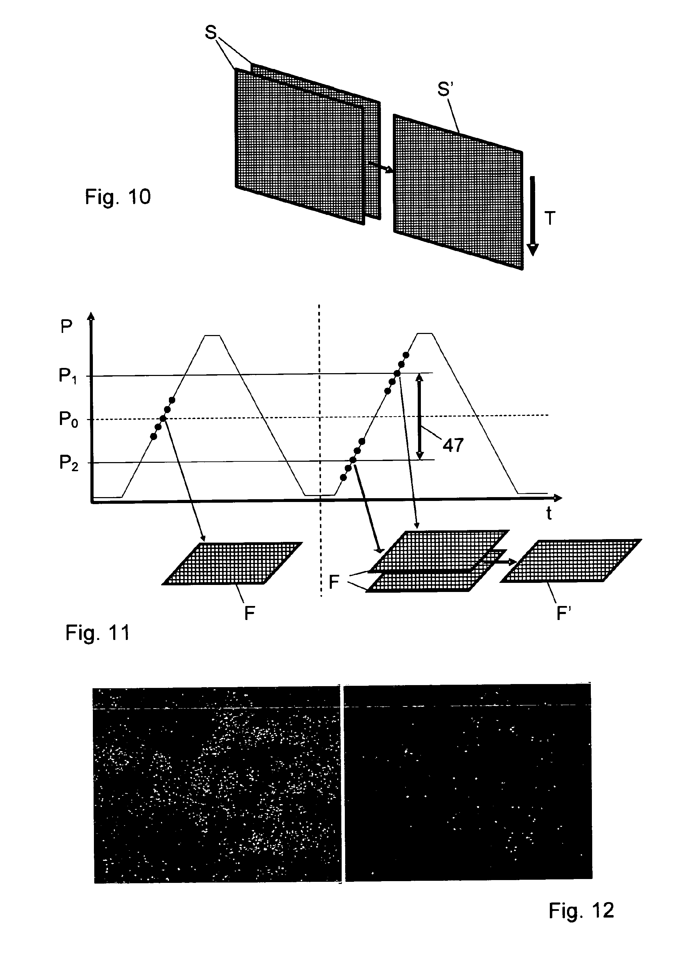

Dies ist anhand von Figur 10 veranschaulicht. Zwei in Richtung der Tiefe T des Objekts aufgenommene Ausgangsbilder S im Form von zwei Tiefenschnitten (vgl. Figur 3) werden durch trilineare Interpolation zu einem Endbild S' zusammengefasst.

Da die beiden Ausgangsbilder S in Form von zwei Tiefenschnitten gleichzeitig und in sehr kurzer Zeit aufgenommen werden, ist sichergestellt, dass eine etwaige Relativbewegung zwischen Sensorkopf und Objekt, insbesondere der menschlichen oder tierischen Haut, während der Aufnahme der beiden zweidimensionalen Ausgangsbilder S keine Rolle spielt.

Im Betriebsmodus 2, bei welchem En-face-Bilder in Echtzeit aufgenommen werden, führt der in einer mittleren Position befindliche Referenzspiegel 16 (siehe Figur 1) nur eine mikroskopische, vorzugsweise oszillierende, Bewegung von ca. +/- 5 µm bis +/- 40 µm durch. Die Position bzw. optische Abbildungseigenschaft des Probenobjektivs 14 ist hierbei vorzugsweise so eingestellt, dass dieses einen Brennpunkt in einer durch makroskopische Verschiebung des Referenzspiegels 16 vorgegebenen mittleren Tiefenposition aufweist. Bei der trilinearen Interpolation der in Echtzeit aufgenommenen En-face-Bilder werden - im Gegensatz zum Betrieb ohne trilineare Interpolation - jeweils zwei Ausgangsbilder in Form von zwei En-face-Bildern an zwei unterschiedlichen Positionen des Referenzspiegels 16 erfasst und zu einem zweidimensionalen Endbild in Form eines En-face-Bildes verrechnet.

Dies ist wird anhand des in Figur 11 gezeigten Diagramms veranschaulicht, welches den Verlauf der Position P des Referenzspiegels 16 über der Zeit t zeigt.

Im linken Teil des Diagramms der Figur 11 ist der Fall ohne trilineare Interpolation dargestellt. Hierbei wird im Betriebsmodus 2 ein zweidimensionales Ausgangsbild F in Form eines Tomogramms aus einer bestimmten Tiefe im Objekt erhalten, indem bei fünf symmetrisch um eine mittlere Position P₀ gelegenen Positionen P des Referenzspiegels 16 gemessen wird.

Im rechten Teil des Diagramms der Figur 11 ist die Anwendung der trilinearen Interpolation veranschaulicht. Zwei zweidimensionale Ausgangsbilder F werden erhalten, indem bei jeweils fünf Positionen P des Referenzspiegels 16 gemessen wird. Die fünf Positionen P liegen jeweils symmetrisch um die Positionen P₁ bzw. P₂, welche vorzugsweise selbst symmetrisch um die mittlere Position P₀ des Referenzspiegels 16 liegen. Der Abstand 47 der Positionen P₁ und P₂ des Referenzspiegels 16 wird hierbei durch die axiale und/oder laterale Pixelgröße 45 bzw. 46 (siehe Figur 9) bestimmt. Bei der bevorzugt symmetrischen Lage der Positionen P₁ und P₂ liegen die entsprechenden Tomogramme F im Objekt jeweils um eine halbe Pixelgröße oberhalb bzw. unterhalb der mittleren Tiefenposition. Die auf diese Weise aufgenommenen beiden Ausgangsbilder F werden dann einer trilinearen Interpolation unterzogen, bei welcher das Endbild F' erhalten wird.

Vorzugsweise wird die Schärfentiefe der optischen Abbildung im Interferometer 10 (siehe Figur 1) so gewählt, dass diese größer ist als die halbe Voxelgröße. Bei einer bevorzugten Voxelgröße von 3 µm muss die Schärfentiefe also größer als 1,5 µm sein.

Da die beschriebene Erfassung der beiden Ausgangsbilder im Betriebsmodus 2 unmittelbar hintereinander erfolgt, typischerweise in einem zeitlichen Abstand von ca. 0,014 Sekunden, ist ein Einfluss einer etwaigen Relativbewegung zwischen Sensorkopf und Objekt, insbesondere der Haut, zwischen der Aufnahme der beiden ursprünglichen Ausgangsbilder auf das erhaltene Ausgangsbild nahezu ausgeschlossen oder vernachlässigbar klein.

Vorzugsweise steht der Sensorkopf bei der Aufnahme der Bilder in direktem Kontakt zur Oberfläche des zu untersuchenden Objekts, insbesondere der Haut, wodurch die Wahrscheinlichkeit einer Relativbewegung stark reduziert wird. Dies ist insbesondere bei Aufnahmen von Bildern der menschlichen oder tierischen Haut von besonderem Vorteil, da diese im Allgemeinen elastisch ist und, insbesondere bei Aufbringung eines Gels, an der Spitze des Sensorkopfes haftet, so dass leichte seitliche Bewegungen oder leichtes Verkippen des Sensorkopfes meist nicht zu einer Relativbewegung zwischen Haut und Sensorkopf führen.

Im Betriebsmodus 3, bei welchem statische dreidimensionale Tomogramme aufgenommen werden, wird - wie oben näher beschrieben - eine Schwebung zwischen der Detektorempfindlichkeitsmodulation einerseits und dem zu erfassenden Interferenzsignal andererseits erzeugt. Dadurch ist der Abstand der einzelnen Abtastpunkte in Tiefenrichtung größer als im Betriebsmodus 1, so dass entsprechend weniger Abtastpunkte, vorzugsweise zwischen 6 und 10, insbesondere 8, zusammengefasst werden, um ein würfelförmiges dreidimensionales Bildelement (Voxel) zu erhalten.

Figur 12 zeigt ein Beispiel eines Ausgangsbildes (links) im Vergleich zu einem entsprechenden Endbild (rechts), welches durch die beschriebene Interpolation erhalten wurde. Das Endbild ist gegenüber dem Ausgangsbild weniger stark verrauscht und erscheint daher weicher" oder "glatter". Bei Vergleichen in der Interpretation der Bilder zu Diagnosezwecken hat sich, insbesondere im Bereich der Dermatologie, herausgestellt, dass den durch trilineare Interpolation erhaltenen Endbildern schneller und sicherer die jeweils relevante diagnostische Information entnommen werden kann. Dies gilt insbesondere für Hohlräume oder Inhomogenitäten mit einer Größe von typischerweise mehr als 10 µm.

Die obigen Ausführungen zur trilinearen Interpolation gelten auch für eine trikubische Interpolation entsprechend, bei welcher die Ausgangswerte nicht durch eine lineare Funktion, sondern durch eine kubische Funktion interpoliert werden.

### 5. System zur optischen Kohärenztomographie

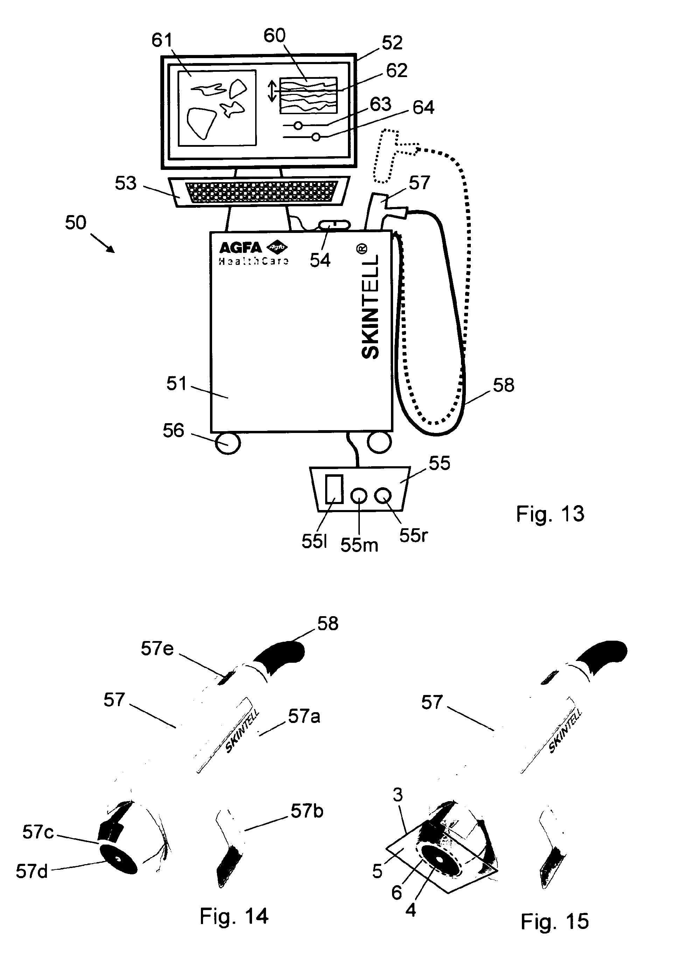

Figur 13 zeigt ein weiteres Beispiel eines Systems 50 zur optischen Kohärenztomographie. Das System 50 umfasst ein Gehäuse 51, Eingabeeinrichtungen in Form einer Tastatur 53, einer Computermaus 54 sowie einer Fußschaltereinrichtung 55, welche einen linken, mittleren und rechten Fußschalter 551, 55m bzw. 55r aufweist. Das Gehäuse 51 ist in dem dargestellten Beispiel fahrbar ausgestaltet, indem es mit Rollen 56 versehen ist.

Ferner ist ein Sensorkopf 57 vorgesehen, welcher über ein Kabel 58 oder einen Kabelschlauch oder -rohr mit dem Gehäuse 51 verbunden ist. In seiner Ruhestellung steckt der Sensorkopf 57 in einer am oder im Gehäuse 51 vorgesehenen Sensorkopfhalterung, aus welcher dieser während der Aufnahme von OCT-Bildern entnommen werden kann, was in der Figur durch den gestrichelt dargestellten Sensorkopf 57 bzw. das gestrichelt dargestellte Kabel 58 angedeutet ist.

Das System umfasst eine Anzeigeeinrichtung 52 in Form eines Flachbildschirms, auf welchem OCT-Bilder 60 und 61, welche durch Aufsetzen des Sensorkopfes 57 auf ein Objekt, insbesondere die Haut eines Patienten, erfasst wurden, dargestellt werden können. In dem in der Figur gezeigten Beispiel handelt es sich bei dem ersten OCT-Bild 60 um einen im Wesentlichen senkrecht zur Oberfläche des untersuchten Objekts verlaufenden Tiefenschnitt, der im oben beschriebenen Betriebsmodus 1 aufgenommen wurde, und bei dem zweiten OCT-Bild 61 um ein zweidimensionales Tomogramm, welches im Wesentlichen parallel zur Oberfläche des untersuchten Objekts verläuft und im oben beschriebenen Betriebsmodus 2 aufgenommen wurde.

Im Bereich des ersten OCT-Bildes 60 wird in der Anzeigeeinrichtung 52 eine Gerade 62 dargestellt, welche in Richtung des angedeuteten Doppelpfeils nach oben bzw. unten verschoben werden kann, beispielsweise indem mit Hilfe der Eingabeeinrichtungen 53, 54 bzw. 55 eine entsprechende Lage der Geraden 62 relativ zum ersten OCT-Bild 60 gewählt wird. Das System 50 ist so konfiguriert, dass entsprechend der gewählten Lage der Geraden 62 im dargestellten ersten OCT-Bild 60 eine senkrecht zum dargestellten ersten OCT-Bild 60 verlaufende Ebene im untersuchten Objekt automatisch ermittelt und dort ein zweidimensionales Tomogramm aufgenommen wird, welches dann als das zweite OCT-Bild 61 dargestellt wird.

Bei dem ersten OCT-Bild 60 handelt es sich vorzugsweise um einen sogenannten Slice, während das zweite OCT-Bild 61 vorzugsweise ein sogenanntes En-face-Bild darstellt, welches in einer der Geraden 62 im ersten OCT-Bild 60 entsprechenden Ebene aufgenommen worden ist.

Am Bildschirm der Anzeigeeinrichtung 52 wird ferner eine Tiefenauswahlanzeige 63 in Form eines entlang einer Geraden beweglichen Schaltersymbols dargestellt, welches die durch eine Wahl der Lage der Geraden 62 relativ zum dargestellten ersten OCT-Bild 60 ausgewählte Tiefe anzeigt. Alternativ oder zusätzlich kann die Tiefe auch in Form von Zahlenwerten angegeben werden.

In der Anzeigeeinrichtung 52 können ferner ein oder mehrere weitere Auswahlanzeigen vorgesehen sein. Im dargestellten Beispiel ist eine Auswahlanzeige 64 vorgesehen, welche eine oder mehrere Eigenschaften des zu untersuchenden Objekts anzeigt. Diese Eigenschaften werden vorzugsweise durch einen Bediener vor der Aufnahme entsprechender OCT-Bilder gewählt und eingegeben. Bei dermatologischen Anwendungen handelt es sich hierbei beispielsweise um einen Parameter zur Charakterisierung der Feuchtigkeit der Haut des jeweiligen Patienten. In der entsprechenden Auswahlanzeige 64 kann ein entsprechendes Schaltersymbol dann entlang einer Geraden kontinuierlich oder in vorgegebenen Schritten zwischen den Positionen "trockene Haut" links und "feuchte Haut" rechts bewegt werden.

In den Sensorkopf 57 ist das in Figur 1 dargestellte Interferometer 10 einschließlich der Optik 28 und des Detektors 30 integriert. Die Lichtquelle 20 einschließlich der eingangsseitigen Optik in Form der beiden Linsen 23 und 24 sind vorzugsweise im Gehäuse 51 des Systems 50 integriert. Der Lichtleiter 26, durch welchen die Lichtquelle 20 einerseits und das Interferometer 10 andererseits miteinander gekoppelt sind, wird in diesem Fall innerhalb des Kabels 58 vom Gehäuse 51 zum Sensorkopf 57 geführt. Im Kabel 58 werden darüber hinaus elektrische Leitungen geführt, welche einerseits zur Energieversorgung und Steuerung des Sensorkopfes 57 dienen und andererseits die bei der Erfassung von OCT-Bildern erzeugten Detektorsignale des Detektors 30 von diesem in das Gehäuse 51 leiten, wo sie einer Verarbeitungseinrichtung (nicht dargestellt) zugeführt werden.

Der in Figur 13 nur stark schematisiert gezeigte Sensorkopf 57 ist in Figur 14 im Detail dargestellt. Im unteren Bereich eines Sensorkopfgehäuses 57a des Sensorkopfes 57 ist ein Griff 57b vorgesehen, durch welchen der Sensorkopf 57 von einer Bedienperson aus der Sensorkopfhalterung am oder im Gehäuse 51 entnommen bzw. wieder in die Sensorkopfhalterung eingesteckt und bei der Aufnahme von OCT-Bildern auf das Objekt aufgesetzt und ggf. an diesem entlang geführt werden kann. Hierbei wird der Sensorkopf 57 mit einer am vorderen Ende des Sensorkopfgehäuses 57a befindlichen Kontaktfläche 57c in Kontakt mit dem zu untersuchenden Objekt, insbesondere der Haut eines Patienten, gebracht.

In der Mitte der Kontaktfläche 57c ist ein Fenster 57d vorgesehen, durch welches Licht aus dem Probenarm 14 des im Sensorkopf 57 befindlichen Interferometers 10 (siehe Figur 1) austreten und dabei das zu untersuchende Objekt bestrahlen kann. Das in unterschiedlichen Tiefen des Objekts reflektierte und/oder zurückgestreute Licht tritt durch das Fenster 57d wieder in den Probenarm 14 des Interferometers 10 ein und kann dort, wie oben bereits ausführlich dargestellt wurde, in Form von Interferenzerscheinungen erfasst und ausgewertet werden.

Am Sensorkopfgehäuse 57a ist ferner eine Statusanzeigeeinrichtung 57e, vorzugsweise in Form einer Leuchtanzeige, vorgesehen, durch welche z.B. die Bereitschaft des Systems 50 und/oder des Sensorkopfes 57 zum Erfassen von OCT-Bildern angezeigt wird.

Im Bereich des hinteren Endes des Sensorkopfgehäuses 57a ist das Kabel 58, das auch als Kabelkanal oder -schlauch ausgebildet sein kann, an den Sensorkopf 57 angeschlossen.

Mit dem oben beschriebenen System 50 zur optischen Kohärenztomographie können drei und zweidimensionale Querschnittsbilder eines Objekts, insbesondere der menschlichen Haut, aufgenommen werden, wobei die Eindringtiefen in die menschliche Haut von bis zu etwa 1 mm erreicht werden können und die Größe der Fläche des untersuchten Hautbereichs typische Abmessungen von etwa 1,8 x 1,5 mm aufweist. Aufgrund der im beschriebenen System 50 verwendeten Infrarotstrahlung mit einer bevorzugten mittleren Wellenlänge von etwa 1,3 µm kann eine Strahlungsbelastung des Patienten, wie beispielsweise bei der Verwendung von Röntgenstrahlung, ausgeschlossen werden. Die mit dem beschriebenen System 50 erfassten OCT-Bilder haben ferner eine hohe Auflösung und erlauben eine Darstellung von einzelnen Objektstrukturen mit einer Größe von bis zu 3 µm. Nicht zuletzt können die mit dem System 50 erfassten OCT-Bilder dazu verwendet werden, die absolute geometrische Ausdehnung der unterschiedlichen Strukturen, d.h. deren Größe, zu messen.

Das System 50 umfasst - wenn auch nicht explizit gezeigt - eine Steuerungseinrichtung zur erfindungsgemäßen Steuerung des Systems 50, insbesondere der optischen Kohärenztomographieeinrichtung, bzw. zur Durchführung der vorstehend und nachfolgend beschriebenen Abläufe. Das System umfasst ferner eine Verarbeitungseinrichtung zur Verarbeitung verschiedener Daten einschließlich der oben beschriebenen Interpolation von Ausgangsbildwerten. Die Steuerungseinrichtung und/oder die Verarbeitungseinrichtung sind vorzugsweise im Gehäuse 51 des Systems 50 integriert.

### 6. Positionierelement zum Positionieren des Sensorkopfes

Wie bereits im Zusammenhang mit Figur 1 erläutert, wird zur Unterstützung der Positionierung des im Sensorkopf 57 integrierten Interferometers 10 relativ zu dem zu untersuchenden Objekt ein Positionierelement 3 am Objekt angebracht. Der Sensorkopf 57 wird dann auf das Positionierelement 3 aufgesetzt und an einer gewünschten Stelle platziert.

Dies ist in Figur 15 anhand eines aus Anschaulichkeitsgründen halbtransparent dargestellten Positionierelements 3 gezeigt. Der Sensorkopf 57 liegt mit der Kontaktfläche 57c (siehe Fig. 14) auf dem Positionierelement 3 auf und ist im dargestellten Beispiel so platziert, dass das Fenster 57d (siehe Fig. 14) des Sensorkopfes 57 im Wesentlichen über einem ersten Bereich 4 des Positionierelements 3 liegt. Der erste Bereich 4 ist für das aus dem Fenster 57d austretende sowie für das vom Objekt (nicht dargestellt) reflektierte und wieder durch das Fenster 57d hindurch tretende Licht im Wesentlichen transparent und vorzugsweise als Durchbruch im Positionierelement 3 ausgestaltet.

Zur Unterstützung einer ersten, vorläufigen Platzierung des Sensorkopfes 57 auf dem Positionierelement 3 ist auf diesem eine kreisförmige Markierung 6 vorgesehen, deren Durchmesser im Wesentlichen dem Außendurchmesser der kreisförmigen Kontaktfläche 57c des Sensorkopfes 57 entspricht. Um eine gute vorläufige Platzierung des Sensorkopfes 57 auf dem Positionierelement 3 zu erreichten, platziert die Bedienperson den Sensorkopf 57 auf dem Positionierelement 3 derart, dass der äußere Rand der Kontaktfläche 57c des Sensorkopfes 57 entlang der kreisförmigen Markierung 6 verläuft.

Der den ersten Bereich 4 umgebende zweite Bereich 5 des Positionierelements 3 unterscheidet sich in seinen optischen Eigenschaften vom ersten Bereich 4 und ist vorzugsweise für das aus dem Sensorkopf 57 austretende sowie für das vom Objekt reflektierte Licht im Wesentlichen undurchlässig. Alternativ oder zusätzlich kann der zweite Bereich 5 Strukturen aufweisen, die sich von den in den OCT-Bildern des Objekts zu erwartenden wiedergegebenen Strukturen unterscheiden. Bei diesen Strukturen kann es sich zum Beispiel um einen oder mehrere Teilbereiche handeln, deren Durchlässigkeit und/oder Reflexionsvermögen für das Licht einen charakteristischen, beispielsweise periodischen, Verlauf aufweist, anhand welchem der zweite Bereich 5, insbesondere ein oder mehrere der Teilbereiche, in den OCT-Bildern erkannt und eindeutig von OCT-Bildbereichen, die dem Objekt entsprechen, unterschieden werden kann. Aufgrund der voneinander verschiedenen optischen Eigenschaften der beiden Bereiche 4 und 5 kann eine Bedienperson in den erfassten und angezeigten OCT-Bildern ohne weiteres erkennen, wenn der Sensorkopf 57 so auf das Positionierelement 3 aufgesetzt worden ist, dass das Fenster 57d des Sensorkopfes 57 nicht exakt über dem transparenten ersten Bereichs 4 zu liegen kommt. Je nachdem, ob der zweite Bereich 5 das Licht absorbiert oder reflektiert, wird der dem zweiten Bereich 5 des Positionierelements 3 entsprechende Bereich in den OCT-Bildern als gleichmäßig dunkler bzw. heller Bereich zu erkennen sein. Anhand dieser Bilder kann dann eine Bedienperson entscheiden, inwieweit die Position des Sensorkopfes 57 gegenüber der bisherigen Position zu verändern ist, um das Fenster 57d möglichst mittig zum ersten Bereich zu platzieren und dadurch zu gewährleisten, dass einerseits OCT-Bilder exakt von dem zu untersuchenden Bereich des Objekts aufgenommen werden und andererseits der jeweils abgebildete Bereich des Objekts eine optimale Größe hat.

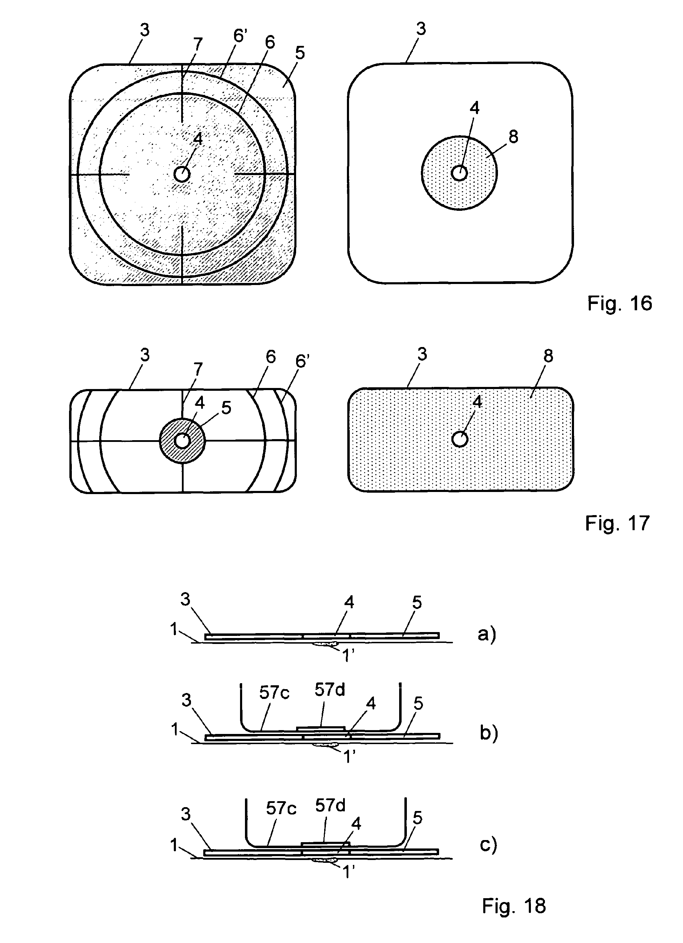

Figur 16 zeigt ein erstes Beispiel für ein Positionierelement 3 in Draufsicht auf die dem Sensorkopf 57 zugewandten Seite (links) und in Draufsicht auf die dem Objekt zugewandten Seite (rechts).

Das Positionierelement 3 besteht aus einer flexiblen Auflage, beispielsweise aus Papier und/oder Kunststoff und/oder Textilmaterial, die eine im Wesentlichen quadratische Grundform mit abgerundeten Ecken aufweist und mit einem Durchbruch versehen ist, welcher den ersten Bereich 4 des Positionierelements 3 bildet. Form und Größe des Durchbruchs entsprechen im Wesentlichen der Form und der Größe des Fensters 57d des Sensorkopfes 57. Im vorliegenden Beispiel ist der Durchbruch daher im Wesentlichen kreisförmig.

Der übrige Teil der Auflage bildet den zweiten Bereich 5 des Positionierelements 3 und ist auf der dem Sensorkopf 57 zugewandten Seite mit einer reflektierenden Schicht versehen, die vom Sensorkopf 57 austretendes Licht reflektiert und vorzugsweise ein Reflexionsvermögen von näherungsweise 100 % aufweist. Die reflektierende Schicht kann vorzugsweise ein oder mehrere Metalle, wie z.B. Aluminium, aufweisen und beispielsweise durch Aufkleben, Aufdrucken oder Aufdampfen eines reflektierenden Materials auf der Auflage erzeugt werden. Alternativ oder zusätzlich ist es auch möglich, die Auflage selbst reflektierend auszugestalten, beispielsweise in Form einer dünnen und/oder flexiblen Metallfolie.

Konzentrisch zum ersten Bereich 4 sind eine erste und zweite kreisförmige Markierung 6 und 6' vorgesehen, die ein Platzieren des Sensorkopfes 57 auf dem Positionierelement 3 unterstützen. Für die erste kreisförmige Markierung 6 gelten die Ausführungen im Zusammenhang mit Fig. 15 entsprechend. Anhand der zweiten kreisförmige Markierung 6' kann das menschliche Auge sogar eine geringfügige exzentrische Platzierung der Kontaktfläche 57c leicht wahrnehmen, was eine zusätzliche Kontrolle der vorläufigen Platzierung des Sensorkopfes 57 auf dem Positionierelement 3 erlaubt.

Zusätzlich zu den kreisförmigen Markierungen 6 und 6' sind gerade Linien 7 als Markierungen vorgesehen, die vorzugsweise wie ein Fadenkreuz um den ersten Bereich 4 herum angeordnet sind und die Platzierung des Sensorkopfes 3 auf dem Positionierelement 3 zusätzlich unterstützen. Die Markierungen 6, 6' und 7 können beispielsweise durch Bedrucken, Gravieren oder Prägen des zweiten Bereichs 5 erzeugt werden.

Die in Fig. 16 (rechts) gezeigte Unterseite der Auflage des Positionierelements 3 weist in einem kreisförmigen Umgebungsbereich des ersten Bereichs 4 einen Haftbereich 8 auf, mittels welchem das Positionierelement 3 am Objekt, insbesondere am menschlichen Hautorgan, lösbar befestigt werden kann. Der Haftbereich wird vorzugsweise durch Beschichtung der Unterseite der Auflage mit Haftklebstoff oder durch Aufkleben eines doppelseitigen Klebebandes erzeugt. Vorzugsweise wird der Haftbereich, insbesondere die Haftklebstoffschicht, mit einer zusätzlichen Schutzschicht (nicht dargestellt) versehen, die erst unmittelbar vor der Verwendung des Positionierelements 3 entfernt wird. Als Schutzschicht wird beispielsweise gewachstes oder silikonisiertes Schutzpapier bevorzugt.

Figur 17 zeigt ein weiteres Beispiel für ein Positionierelement 3 in Draufsicht auf die dem Sensorkopf 57 zugewandten Seite (links) und in Draufsicht auf die dem Objekt zugewandten Seite (rechts). Im Unterschied zu dem in Fig. 16 gezeigten ersten Beispiel ist die dem Sensorkopf 57 zugewandte Seite der Auflage lediglich in einem den ersten Bereich 4 ringförmig umgebenden Bereich, welcher in diesem Beispiel den zweiten Bereich 5 des Positionierelements 6 bildet, mit einer reflektierenden Schicht versehen. Ferner ist in diesem Beispiel die Rückseite der Auflage (Fig. 17 rechts) vollständig mit einem Haftbereich versehen. Im Übrigen gelten für das in Fig. 17 gezeigte Beispiel die Ausführungen im Zusammenhang mit dem in Fig. 16 gezeigten ersten Beispiel entsprechend.

Das erfindungsgemäße Verfahren wird nachfolgend anhand von Figur 18, welche drei Querschnitte durch Objekt 1, Positionierelement 3 und Sensorkopf 57 zeigt, näher erläutert.

Teil a) von Figur 18 zeigt das Positionierelement 3, das derart auf dem Objekt 1, in diesem Fall die menschliche Haut, aufgebracht, insbesondere aufgeklebt ist, dass der erste Bereich 4 über einem interessierenden und näher zu untersuchenden Bereich 1' des Objekts 1, beispielsweise eine Hautläsion, liegt. Aus Anschaulichkeitsgründen sind Positionierelement 3 und Objekt 1 in der hier gewählten Darstellung beabstandet gezeigt, obwohl das Positionierelement 3 am Objekt 1 anliegt.

Teil b) von Figur 18 zeigt den mit der Kontaktfläche 57c auf dem Positionierelement 3 aufliegenden Sensorkopf, welcher durch die Bedienperson, insbesondere mit Hilfe von Markierungen 6, 6' und 7 (siehe Fig. 15 bis 17), auf dem Positionierelement 3 vorläufig platziert worden ist, so dass das Fenster 57d des Sensorkopfes im Bereich des ersten Bereichs 4 des Positionierelements 3 zu liegen kommt. Vorzugsweise befindet sich zwischen Kontaktfläche 57c und Fenster 57d des Sensorkopfes einerseits und Positionierelement 3 und Objekt 1 andererseits ein Medium (sog. Index Matching Gel), durch welches die optischen Brechungsindizes aneinander angepasst werden, um Lichtverluste aufgrund von Reflexionen an Grenzflächen zu minimieren.

Um eine noch genauere Positionierung des Fensters 57d des Sensorkopfes gegenüber dem Objekt 1, insbesondere gegenüber dem zu untersuchenden Bereich 1', zu erreichen, bei welcher das Fenster 57d exakt über dem ersten Bereich 4 liegt, werden in erfindungsgemäßer Weise OCT-Bilder herangezogen, anhand welcher die Bedienperson erkennen kann, wenn das Fenster 57d nicht exakt über dem ersten Bereich 4 platziert ist.

Bei dem in Teil b) von Fig. 18 dargestellten Beispiel ist das Fenster 57d gegenüber dem ersten Bereich 4 etwas nach links zum zweiten Bereich 5 hin verschoben, so dass der in das Bildfeld ragende Teil des zweiten Bereichs 5 aufgrund seiner vom ersten Bereich 4 abweichenden optischen Eigenschaften in den entsprechenden OCT-Bildern zu erkennen sein wird. Insbesondere wenn die OCT-Bilder in Echtzeit aufgenommen und wiedergegeben werden, kann die Bedienperson dann durch allmähliches Verrücken des Sensorkopfes nach rechts bei gleichzeitiger Beobachtung der jeweils erhaltenen OCT-Bilder schließlich den Sensorkopf so platzieren, dass dieser - wie in Teil c) von Fig. 18 dargestellt - exakt über dem ersten Bereich 4 des Positionierelements 3 liegt und dadurch bezüglich des zu untersuchenden Bereichs 1' des Objekts 1 optimal positioniert ist.

Im Folgenden werden beispielhaft einige bei dem beschriebenen Positionierverfahren aufgenommene und wiedergegebene OCT-Bilder näher erläutert.

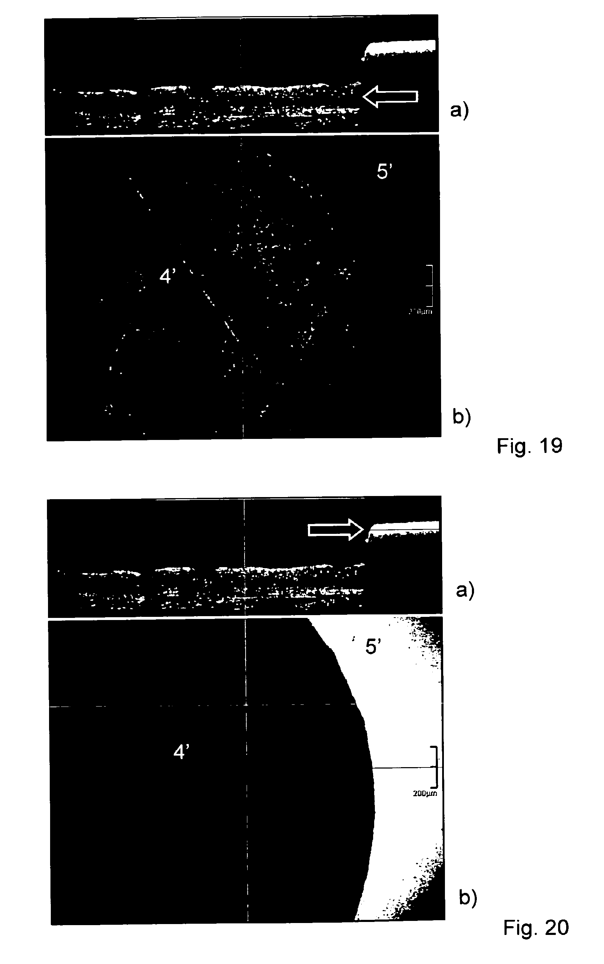

Die Figuren 19 und 20 zeigen OCT-Bilder, die mit einem Positionierelement 3 erhalten wurden, dessen zweiter Bereich 5 das vom Sensorkopf ausgegebene bzw. vom Objekt 1 reflektierte Licht größtenteils absorbiert und im Wesentlichen nicht reflektiert.

Bei Teil a) der Figuren handelt es sich jeweils um einen im ersten Betriebsmodus aufgenommenen zweidimensionalen Tiefenschnitt (sog. Slice, siehe Fig. 3) durch Positionierelement (oben) und Objekt (unten). Bei Teil b) der Figuren handelt es sich jeweils um ein im zweiten Betriebsmodus in einer bestimmten Tiefe im Objekt bzw. Positionierelement aufgenommenes zweidimensionales Tomogramm (sog. En-face-Bild, siehe Fig. 4). Die eingezeichneten Pfeile zeigen im jeweiligen Slice die Tiefe an, in welcher das zugehörige En-face-Bild aufgenommen worden ist.

Wie aus den Figuren 19 und 20 ersichtlich ist, ist in den in unterschiedlichen Tiefen erhaltenen En-face-Bildern der dem zweiten Bereich 5 des Positionierelements 3 entsprechende zweite Bildbereich 5' jeweils deutlich von dem ersten Bildbereich 4', welcher dem ersten Bereich 4 des Positionierelements 3 entspricht, zu unterscheiden. In Fig. 19 zeigt der erste Bildbereich 4' Strukturen in einer bestimmten Tiefe des Objekts, während der zweite Bildbereich 5' aufgrund des für Licht undurchlässigen zweiten Bereichs 5 gleichmäßig schwarz wiedergegeben wird. In Fig. 20 wird der erste Bildbereich 4' gleichmäßig schwarz wiedergegeben, da sich in dieser Tiefe im ersten Bereich 4 Luft bzw. Index-Matching-Gel befindet, welche bzw. welches keine relevante Lichtstreuung verursacht. Aufgrund von, wenn auch nur geringfügigen, Lichtreflexionen im daran angrenzenden zweiten Bereich 5 ist der zweite Bildbereich 5' im En-face-Bild vergleichsweise hell.

Wie bereits oben ausführlich beschrieben, kann eine Bedienperson anhand dieser, vorzugsweise in Echtzeit aufgenommenen, OCT-Bilder auf einfache und bequeme Weise den Sensorkopf relativ zum Positionierelement 6 verschieben und dabei so platzieren, dass der vom Objekt erfasste erste Bildbereich 4' optimal ist, während der vom zweiten Bereich 5 erhaltene zweite Bildbereich 5' minimiert oder aus den OCT-Bildern ganz eliminiert wird.

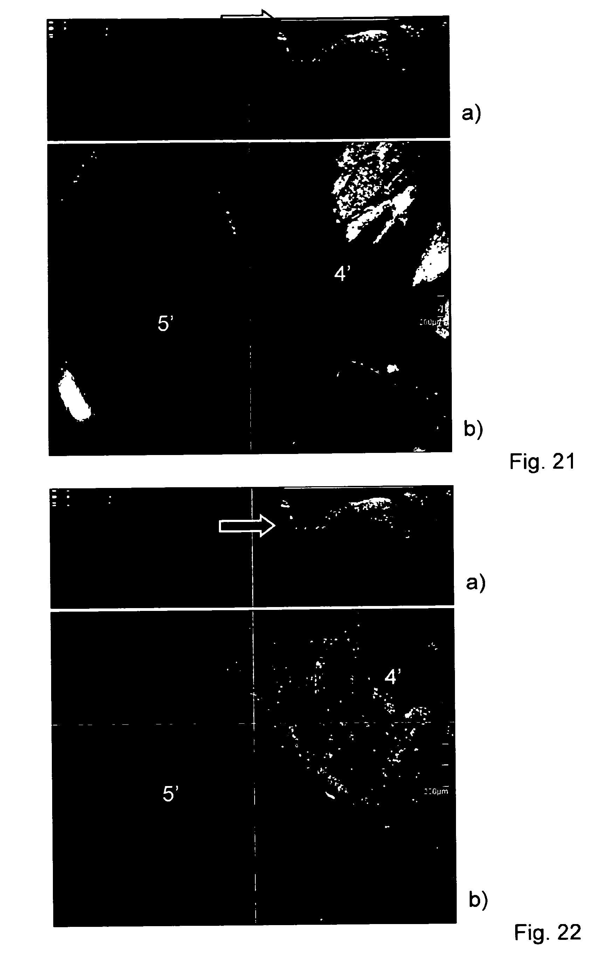

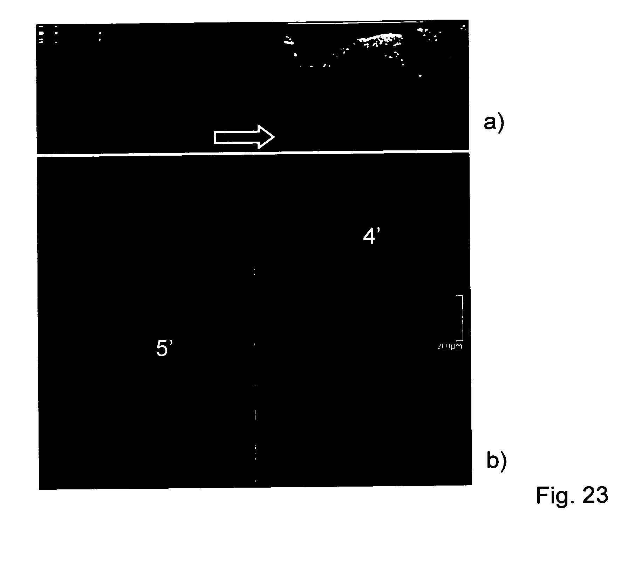

Die Figuren 21 bis 23 zeigen OCT-Bilder, die mit einem Positionierelement 3 erhalten wurden, dessen zweiter Bereich 5 das vom Sensorkopf ausgegebene bzw. vom Objekt 1 reflektierte Licht größtenteils reflektiert. Bezüglich der Figurenteile a) und b) gelten die obigen Ausführungen zu den Figuren 19 und 20 entsprechend.

Bei der Aufnahme der in diesem Beispiel gezeigten OCT-Bilder wurde der Detektor des OCT-Systems so betrieben und/oder angesteuert, dass dieser bei Erfassung des vom zweiten Bereich 5 des Positionierelement 3 reflektierten Lichts in Sättigung ist, d.h. die von Detektor jeweils gelieferten Detektorsignale einen vorgegebenen oder vorgebbaren Maximalwert einnehmen. Bei der Demodulation, bei welcher aus den Detektorsignalen der erfassten Interferenzbilder die OCT-Bilder abgeleitet werden, werden nur Änderungen des Detektorsignals erfasst. Da der Detektor im Sättigungsbetrieb jedoch keine Änderung des Detektorsignals zeigt, entsteht nach der Demodulation im jeweiligen OCT-Bild ein völlig schwarzer und damit rauschfreier zweiter Bildbereich 5', welcher dem zweiten Bereich 5 des Positionierelements 3 entspricht. In den gezeigten En-face-Bildern ergibt sich daher in allen Tiefen ein hoher Kontrast zwischen den ersten und zweiten Bildbereichen 4' bzw. 5'.

Nachfolgend werden weitere Aspekte, Alternativen und Vorteile des Positionierelements näher erläutert.

Vorzugsweise hat das Positionierelement ein kleines Loch in der Mitte, das rund oder eckig ausgebildet sein kann. Der Durchmesser bzw. die Kantelänge des Loches ist vorzugsweise an die Bildfelddiagonale (BFD) des OCT-Systems angepasst, wobei der Durchmesser etwa 100 % bis 200 % der BFD bei rundem Loch bzw. die Kantenlänge 75 % bis 170 % der BFD bei eckigem Loch beträgt.

Vorzugsweise ist das Material des Positionierelements so gewählt bzw. beschaffen, dass es einen sichtbaren Kontrast in möglichst allen Tiefen erzeugt. Dies kann z.B. durch Abschattung erfolgen, wobei das Material nicht transparent für das verwendete Licht, insbesondere bei einer Wellenlänge von etwa 1,3 µm, ist. Ferner kann dies durch eine starke Reflexion erfolgen, wobei das Material bei etwa 1,3 µm stark reflektiert. Die Reflexion ist dabei vorzugsweise so stark, dass der Detektor in den Sättigungsbetrieb übergeht. Dies führt nach einer Demodulation (FSA) der Detektorsignale im Bereich des stark reflektierenden Materials zu einem völlig schwarzen Bild ohne Rauschen.

Der Übergang Loch zu Positionierelement ist sowohl im Slice- als auch im En-face-Bild sichtbar. Insbesondere bei Verwendung von reflektierendem Material trifft gilt dies sogar in allen Tiefen, in welchen OCT-Bilder aufgenommen werden.

Der klebende Bereich auf der Rückseite des Positionierelements kann, ggf. deutlich, kleiner sein als die Gesamtfläche des Positionierelements und muss auf der Haut nur leicht kleben. Dies ermöglicht ein einfaches und schmerzloses Abziehen des Positionierelements.

Der Außendurchmesser des Positionierelements kann größer sein als die Spitze des Sensorkopfes und zum einfachen Auffinden der Mitte z.B. ein Fadenkreuz und Zentrierkreise aufweisen.

Der Außendurchmesser des Positionierelements kann, ggf. deutlich, kleiner sein als die Spitze, insbesondere die Auflagefläche, des Sensorkopfes.

Der Benutzer positioniert die Öffnung des Positionierelements exakt auf die Stelle der Haut, die mit OCT-System untersucht werden soll. Der Sensorkopf wird nun so über das oder auf dem Positionierelement platziert, dass der innere der konzentrischen Zentrierkreise exakt abgedeckt wird. Der äußere der Kreise dient als zusätzliche Positionierkontrolle, da das menschliche Auge leicht Ungenauigkeiten bezüglich Konzentrizität wahrnimmt und so dem Benutzer eine einfache Korrektur der Positionierung des Sensorkopfs ermöglicht.

Das auf dem Positionierelement vorzugsweise aufgedruckte Fadenkreuz ermöglicht eine zusätzlich Positionskontrolle des Sensorkopfs.

Dadurch, dass das Positionierelement einen sichtbaren Kontrast in den OCT-Bildern erzeugt, kann der Benutzer die gesuchte Hautstelle sicher und bequem auffinden. Dabei ergeben sich keine Ungenauigkeiten durch Toleranzen.

Die Benutzung des Positionierelements ist nahezu selbsterklärend und benötigt in der Regel keine aufwändige Schulung des Benutzers. Aufgrund des beschriebenen Prinzips ist mit dieser Lösung eine sichere Positionierung des Sensorkopfs auf der ROI (Region of Interest) möglich und damit gewährleistet, dass das erstellte Bild der entsprechenden Hautregion entstammt. Das System ist a priori "toleranzfrei", da ein OCT-Bild nur im Bereich der Öffnung entsteht.

Durch das reflektierende Material des Positionierelements ist außerdem gewährleistet, dass die abgedeckten Regionen der Haut im OCT-Bild ausgeblendet werden und sich ein guter Kontrast zwischen dem Bereich auf dem Positionierelement und der Haut in allen Tiefen ergibt.

Das Positionierelement ist ferner einfach aufgebaut und kann preisgünstig hergestellt werden. Für das Positionierelement können biokompatible Materialien verwendet werden, die Hautirritationen vermeiden. Die Klebefläche kann so gewählt werden, dass ein leichtes Abziehen des Positionierelements möglich ist, ohne die Haut dabei zu verletzen. Mit einem kleinen Positionierelement können auch schwer zugängliche Hautregionen markiert werden.

### 7. Weitere erfinderische Aspekte des Systems bzw. Verfahrens

Das vorstehend näher beschriebene System bzw. Verfahren zur OCT weist einzelne Merkmale oder Merkmalskombinationen auf, durch die das System bzw. Verfahren in der Handhabung und Bilderfassung einfacher, schneller und zuverlässiger gemacht wird, ohne dass hierbei zwingend alle in den unabhängigen Ansprüchen aufgeführten Merkmale erforderlich sind. Diese Merkmale bzw. Merkmalskombinationen werden ebenfalls als Erfindung angesehen.

Als Erfindung wird insbesondere ein System zur optischen Kohärenztomographie angesehen mit mindestens einem Interferometer zur Ausgabe von Licht, mit dem ein Objekt bestrahlt wird, und einem Detektor zur Erfassung von Licht, welches vom Objekt reflektiert und/oder zurückgestreut wird, wobei das System durch ein oder mehrere Merkmale gekennzeichnet ist, die vorstehend, insbesondere in den Abschnitten 1 bis 6 und/oder im Zusammenhang mit den Figuren, näher beschrieben wurden. Das diesem System entsprechende Verfahren wird ebenfalls als Erfindung angesehen.

## Patentansprüche

1. System zur optischen Kohärenztomographie mit
- einem Interferometer (10) und einem Sensorkopf (57), über welchen elektromagnetische Strahlung vom Interferometer (10) an ein zu untersuchendes Objekt (1) ausgegeben und vom Objekt (1) reflektierte elektromagnetische Strahlung in das Interferometer (10) zurück geführt werden kann,
- einem Positionierelement (3) zum Positionieren des Sensorkopfes (57) relativ zu dem zu untersuchenden Objekt (1) mit einer Auflage, welche am Objekt (1) angeordnet und auf welcher der Sensorkopf (57) platziert werden kann, und einem ersten Bereich (4), welcher für die vom Interferometer (10) ausgegebene und vom Objekt (1) reflektierte Strahlung im Wesentlichen durchlässig ist, und einem zweiten Bereich (5), dessen Durchlässigkeit für die vom Interferometer (10) ausgegebene und/oder vom Objekt (1) reflektierte Strahlung von der Durchlässigkeit des ersten Bereichs (4) abweicht,
- einer Bilderzeugungseinrichtung (51) zur Erzeugung eines oder mehrerer Bilder (60, 61) anhand der vom Objekt (1) und/oder vom Positionierelement (3), insbesondere vom zweiten Bereich (5) des Positionierelements (3), reflektierten elektromagnetischen Strahlung,
- einer Anzeigeeinrichtung (52) zur Wiedergabe der erzeugten Bilder (60, 61) und
- einer Steuerungseinrichtung (51) zur Steuerung der Erzeugung und Wiedergabe der Bilder (60, 61) in der Weise, dass der Sensorkopf (57) anhand der erzeugten und wiedergegebenen Bilder (60, 61) in eine gewünschte Position auf dem Positionierelement (3) gebracht werden kann.

2. System nach Anspruch 1, wobei der zweite Bereich (5) für die vom Interferometer (10) ausgegebene und/oder vom Objekt (1) reflektierte Strahlung im Wesentlichen undurchlässig ist.

3. System nach Anspruch 1 oder 2, wobei der zweite Bereich (5) eine Struktur aufweist, deren optische Eigenschaften sich von den optischen Eigenschaften von, insbesondere typischen, Strukturen des Objekts (1) unterscheiden.

4. System nach einem der vorangehenden Ansprüche, wobei die Auflage biegsam ausgestaltet ist und sich insbesondere an Konturen des Objekts (1) anpassen kann.

5. System nach einem der vorangehenden Ansprüche, wobei die Auflage mit dem Objekt (1) in Kontakt gebracht, insbesondere lösbar am Objekt (1) fixiert, werden kann.

6. System nach einem der vorangehenden Ansprüche, wobei der erste Bereich (4) der Auflage als Durchbruch in der Auflage ausgebildet ist.

7. System nach einem der vorangehenden Ansprüche, wobei die Form und/oder Größe des ersten Bereichs (4) der Auflage an die Form bzw. Größe eines Durchtrittsbereichs (57d) des Sensorkopfes (57), durch welchen die vom Interferometer (10) ausgegebene und die vom Objekt (1) reflektierte Strahlung hindurch treten kann, angepasst ist.

8. System nach einem der vorangehenden Ansprüche, wobei am Positionierelement (3) ein oder mehrere Markierungen (6, 6', 7) vorgesehen sind, welche ein Positionieren des Sensorkopfes (57) relativ zum Positionierelement (3), insbesondere relativ zum ersten und/oder zweiten Bereich (4 bzw. 5) der Auflage, unterstützen.

9. System nach einem der vorangehenden Ansprüche, wobei der zweite Bereich (5) der Auflage eine Durchlässigkeit für die vom Interferometer (10) ausgegebene bzw. vom Objekt (1) reflektierte Strahlung von weniger als 10⁻⁴, insbesondere weniger als 10⁻⁶, aufweist.

10. System nach einem der vorangehenden Ansprüche, wobei der zweite Bereich (5) der Auflage eine Reflexionsschicht aufweist, welche die vom Interferometer (10) ausgegebene Strahlung reflektiert.

11. System nach einem der vorangehenden Ansprüche, wobei der zweite Bereich (5) der Auflage, insbesondere die Reflexionsschicht, ein Reflexionsvermögen für die vom Interferometer ausgegebene Strahlung von mindestens 30 %, insbesondere mindestens 50 %, aufweist.

12. System nach einem der vorangehenden Ansprüche, wobei das System derart ausgebildet ist, dass Bilder (60, 61) aus unterschiedlichen Tiefen des Objekts (1) anhand der in den unterschiedlichen Tiefen des Objekts (1) reflektierten Strahlung erzeugt und wiedergegeben werden, wobei in den aus den unterschiedlichen Tiefen des Objekts (1) erhaltenen und wiedergegebenen Bildern (60, 61) ein Übergang zwischen Objekt (1) und Positionierelement (3), insbesondere zwischen dem ersten und zweiten Bereich (4 bzw. 5) des Positionierelements (3), erkennbar ist.

13. System nach einem der vorangehenden Ansprüche mit einem Detektor (30) zur Erfassung der vom Objekt (1) und/oder vom Positionierelement (3) reflektierten und in das Interferometer (10) zurückgeführten Strahlung, wobei die Steuereinrichtung zur Ansteuerung des Detektors (30) derart ausgebildet ist, dass sich dieser bei der Erfassung der vom Positionierelement (3) reflektierten und in das Interferometer (10) zurückgeführten Strahlung in einem Sättigungsbetrieb befindet.

14. System nach einem der vorangehenden Ansprüche, wobei
- die Bilderzeugungseinrichtung (51) zur Erzeugung von Bildern (60, 61) des Objekts (1) bzw. Positionierelements (3) mit einer Rate von mehr als einem Bild pro Sekunde, insbesondere mehr als fünf Bildern pro Sekunde, ausgebildet ist und/oder
- die Anzeigeeinrichtung (52) zur Wiedergabe von Bildern (60, 61) des Objekts (1) bzw. Positionierelements (3) mit einer Rate von mehr als einem Bild pro Sekunde, insbesondere mehr als fünf Bildern pro Sekunde, ausgebildet ist.

15. Positionierelement (3) zur Verwendung in einem System zur optischen Kohärenztomographie mit einem Interferometer (10) und einem Sensorkopf (57), über welchen elektromagnetische Strahlung vom Interferometer (10) an ein zu untersuchendes Objekt (10) ausgegeben und vom Objekt (1) reflektierte elektromagnetische Strahlung in das Interferometer (10) zurück geführt werden kann, wobei das Positionierelement (3)
- ein Positionieren des Sensorkopfes (57) relativ zu einem zu untersuchenden Objekt (1) erlaubt und
- eine Auflage aufweist, welche am Objekt (1) angeordnet werden kann und auf welcher der Sensorkopf (57) platziert werden kann, wobei die Auflage einen ersten Bereich (4), welcher für die vom Interferometer (10) ausgegebene und vom Objekt (1) reflektierte Strahlung im Wesentlichen durchlässig ist, und einen zweiten Bereich (5), dessen Durchlässigkeit für die vom Interferometer (10) ausgegebene und/oder vom Objekt (1) reflektierte Strahlung von der Durchlässigkeit des ersten Bereichs (4) abweicht, aufweist.

16. Verfahren zur optischen Kohärenztomographie, bei welchem
- elektromagnetische Strahlung von einem Interferometer (10) über einen Sensorkopf (57) an ein zu untersuchendes Objekt (1) ausgegeben und vom Objekt (1) reflektierte elektromagnetische Strahlung über den Sensorkopf (57) in das Interferometer (10) zurück geführt wird,
- ein Positionierelement (3) zum Positionieren des Sensorkopfes (57) relativ zu dem zu untersuchenden Objekt (1) am Objekt (1) angeordnet wird und der Sensorkopf (57) auf der Auflage platziert wird, wobei die Auflage einen ersten Bereich (4), welcher für die vom Interferometer (10) ausgegebene und vom Objekt (1) reflektierte Strahlung im Wesentlichen durchlässig ist, und einen zweiten Bereich (5), dessen Durchlässigkeit für die vom Interferometer (10) ausgegebene und/oder vom Objekt (1) reflektierte Strahlung von der Durchlässigkeit des ersten Bereichs (4) abweicht, aufweist,
- anhand der vom Objekt (1) und/oder vom Positionierelement (3), insbesondere vom zweiten Bereich (5) des Positionierelements (3), reflektierten elektromagnetischen Strahlung ein oder mehrere Bilder (60, 61) erzeugt und wiedergegeben werden und
- der Sensorkopf (57) anhand der erzeugten und wiedergegebenen Bilder (60, 61) in eine gewünschte Position auf dem Positionierelement (3) gebracht wird.
